# EUROPEAN PATENT APPLICATION

(11) **EP 3 246 415 A1**
(43) Date of publication of application: **22.11.2017**
(21) Application number: 16382219.0
(22) Date of filing: 18.05.2016
(51) Int. Cl.: C12Q 1/68

(54) **METHODS FOR THE PROGNOSIS OF PROSTATE CANCER**

(71) Applicant: Asociación Centro de Investigación Cooperativa en Biociencias - CIC bioGUNE, 48160 Derio (ES); Fundacion Ikerbasque / Ikerbasque Fundazioa, 48013 Bibao (ES)
(72) Inventor: CARRACEDO PÉREZ, Arkaitz, E-48006 Bilbao (ES); TORRANO MOYA, Verónica, E-39770 Laredo (ES)
(74) Representative: ABG Patentes, S.L.

(57) **Abstract**

The present invention relates to methods useful for predicting the outcome of a patient suffering from prostate cancer or for predicting the response to therapy of a patient suffering from prostate cancer, which comprise the determination of the expression level of PGC1A or of a gene signature under the control of PGC1A. These methods can be used to identify those patients who are at a high risk of recurrence or metastasis. The identification of this subgroup of patients may guide the selection of therapies, improving financial and health outcomes. Moreover, the expression levels of these genes can also be used for the selection of a patient to be treated for advanced, recurrent or metastatic prostate cancer. Further, the invention also relates to kits and reagents to determine the expression levels of these genes.

SEQUENCE LISTING

<110>  ASOCIACIÓN CENTRO DE INVESTIGACIÓN COOPERATIVA EN
       BIOCIENCIAS - CIC bioGUNE
       FUNDACIÓN IKERBASQUE/ IKERBASQUE FUNDAZIOA

<120>  METHODS FOR THE PROGNOSIS OF PROSTATE CANCER

<130>  P13005EP00

<160>  64

<170>  PatentIn version 3.5

<210>  1
<211>  20
<212>  DNA
<213>  Artificial Sequence

<220>
<223>  qRT-PCR primer

<400>  1
cagacgctac aggtcccaac                                                  20


<210>  2
<211>  20
<212>  DNA
<213>  Artificial Sequence

<220>
<223>  qRT-PCR reverse sequence

<400>  2
ctgtccactc cactgtcagg                                                  20


<210>  3
<211>  20
<212>  DNA
<213>  Artificial Sequence

<220>
<223>  qRT-PCR primer

<400>  3
tgaatctcac gcgcctacta                                                  20


<210>  4
<211>  20
<212>  DNA
<213>  Artificial Sequence

<220>
<223>  qRT-PCR primer

<400>  4
ttgtgttctc ggcctctctt                                                  20


<210>  5

<211>  19
<212>  DNA
<213>  Artificial Sequence

<220>
<223>  qRT-PCR primer

<400>  5
aggagccatt gatgtgtgc                                                    19


<210>  6
<211>  24
<212>  DNA
<213>  Artificial Sequence

<220>
<223>  qRT-PCR primer

<400>  6
ctgctttggt ctttatacca gcta                                              24


<210>  7
<211>  23
<212>  DNA
<213>  Artificial Sequence

<220>
<223>  qRT-PCR primer

<400>  7
gatccccaaa aagtgaatat caa                                               23


<210>  8
<211>  19
<212>  DNA
<213>  Artificial Sequence

<220>
<223>  qRT-PCR primer

<400>  8
atcctggctc cagacatcc                                                    19


<210>  9
<211>  20
<212>  DNA
<213>  Artificial Sequence

<220>
<223>  qRT-PCR primer

<400>  9
agggaagttt gccagtgaga                                                   20


<210>  10
<211>  22
<212>  DNA
<213>  Artificial Sequence

<220>
<223>  qRT-PCR primer

<400>  10
ttcaccacca catctggttt ac                                                22


<210>  11
<211>  23
<212>  DNA
<213>  Artificial Sequence

<220>
<223>  qRT-PCR primer

<400>  11
gtgtagactc catctcctgc act                                               23


<210>  12
<211>  20
<212>  DNA
<213>  Artificial Sequence

<220>
<223>  qRT-PCR primer

<400>  12
tgtggttgta agggaacacg                                                   20


<210>  13
<211>  24
<212>  DNA
<213>  Artificial Sequence

<220>
<223>  qRT-PCR primer

<400>  13
tgtactgtac tgaagcccac actt                                              24


<210>  14
<211>  22
<212>  DNA
<213>  Artificial Sequence

<220>
<223>  qRT-PCR primer

<400>  14
ttcatctctt ggactttgct ca                                                22


<210>  15
<211>  22
<212>  DNA
<213>  Artificial Sequence

<220>
<223>  qRT-PCR primer

<400>  15
gaaattcaca gcatgcaatc ag                                                22


<210>  16
<211>  21
<212>  DNA
<213>  Artificial Sequence

<220>
<223>  qRT-PCR primer

<400>  16
tctacttgga ggaacgctca a                                                 21


<210>  17
<211>  19
<212>  DNA
<213>  Artificial Sequence

<220>
<223>  qRT-PCR primer

<400>  17
cggtggcagt tggtttaag                                                    19


<210>  18
<211>  20
<212>  DNA
<213>  Artificial Sequence

<220>
<223>  qRT-PCR primer

<400>  18
agcatcactt ggatggttcc                                                   20


<210>  19
<211>  19
<212>  DNA
<213>  Artificial Sequence

<220>
<223>  qRT-PCR primer

<400>  19
gcccagaaca ctgttacgc                                                    19


<210>  20
<211>  22
<212>  DNA
<213>  Artificial Sequence

<220>
<223>  qRT-PCR primer

<400>  20
tgcagcttca ataccctttta gc                                               22

<210>  21
<211>  19
<212>  DNA
<213>  Artificial Sequence

<220>
<223>  qRT-PCR primer

<400>  21
cccgggataa ggacaagag                                                    19


<210>  22
<211>  21
<212>  DNA
<213>  Artificial Sequence

<220>
<223>  qRT-PCR primer

<400>  22
catctgcttc ttctgcaaac c                                                 21


<210>  23
<211>  22
<212>  DNA
<213>  Artificial Sequence

<220>
<223>  qRT-PCR primer

<400>  23
gcacagatca tcaagattgg ac                                                22


<210>  24
<211>  26
<212>  DNA
<213>  Artificial Sequence

<220>
<223>  qRT-PCR primer

<400>  24
ttgttgaaca taaccactaa attcct                                            26


<210>  25
<211>  20
<212>  DNA
<213>  Artificial Sequence

<220>
<223>  qRT-PCR primer

<400>  25
gcaccccaat gatcatgtta                                                   20


<210>  26

<211>  20
<212>  DNA
<213>  Artificial Sequence

<220>
<223>  qRT-PCR primer

<400>  26
attgctgtgg gaaaggtgtc                                                   20


<210>  27
<211>  20
<212>  DNA
<213>  Artificial Sequence

<220>
<223>  qRT-PCR primer

<400>  27
agctgtgctt ctcgtcttgc                                                   20


<210>  28
<211>  20
<212>  DNA
<213>  Artificial Sequence

<220>
<223>  qRT-PCR primer

<400>  28
agattgcaca cctcctaccc                                                   20


<210>  29
<211>  20
<212>  DNA
<213>  Artificial Sequence

<220>
<223>  qRT-PCR primer

<400>  29
tgctacagcc ctgactttga                                                   20


<210>  30
<211>  23
<212>  DNA
<213>  Artificial Sequence

<220>
<223>  qRT-PCR primer

<400>  30
tgatcttgtc tccaacaaac cat                                               23


<210>  31
<211>  19
<212>  DNA
<213>  Artificial Sequence

<220>
<223>  qRT-PCR primer

<400>  31
caccctcact gcgctagac                                                    19


<210>  32
<211>  24
<212>  DNA
<213>  Artificial Sequence

<220>
<223>  qRT-PCR primer

<400>  32
ttcttcactt tgtcattcaa tcct                                              24


<210>  33
<211>  19
<212>  DNA
<213>  Artificial Sequence

<220>
<223>  qRT-PCR primer

<400>  33
tttttgatgc tgccaaagc                                                    19


<210>  34
<211>  20
<212>  DNA
<213>  Artificial Sequence

<220>
<223>  qRT-PCR primer

<400>  34
ttcctccagg tccttgaatg                                                   20


<210>  35
<211>  20
<212>  DNA
<213>  Artificial Sequence

<220>
<223>  qRT-PCR primer

<400>  35
gaggttttgc tggtggtgtt                                                   20


<210>  36
<211>  20
<212>  DNA
<213>  Artificial Sequence

<220>
<223>  qRT-PCR primer

<400>  36
tgaaatttct gggccaattc                                                   20


<210>  37
<211>  21
<212>  DNA
<213>  Artificial Sequence

<220>
<223>  qRT-PCR primer

<400>  37
aacacagata tcgccaagga g                                                 21


<210>  38
<211>  20
<212>  DNA
<213>  Artificial Sequence

<220>
<223>  qRT-PCR primer

<400>  38
attgcatctt cagccagcat                                                   20


<210>  39
<211>  20
<212>  DNA
<213>  Artificial Sequence

<220>
<223>  qRT-PCR primer

<400>  39
ctggtggcag tcagagaatg                                                   20


<210>  40
<211>  18
<212>  DNA
<213>  Artificial Sequence

<220>
<223>  qRT-PCR primer

<400>  40
cagcagggcg aaatgtct                                                     18


<210>  41
<211>  19
<212>  DNA
<213>  Artificial Sequence

<220>
<223>  qRT-PCR primer

<400>  41
gcggcttatc aaacacgag                                                    19

<210>  42
<211>  26
<212>  DNA
<213>  Artificial Sequence

<220>
<223>  qRT-PCR primer

<400>  42
agggaggcta tttataatga aatctg                                            26


<210>  43
<211>  23
<212>  DNA
<213>  Artificial Sequence

<220>
<223>  qRT-PCR primer

<400>  43
aaaattggag tctgtttgct gtt                                               23


<210>  44
<211>  24
<212>  DNA
<213>  Artificial Sequence

<220>
<223>  qRT-PCR primer

<400>  44
tgtgctttag cttttagttc ttgg                                              24


<210>  45
<211>  20
<212>  DNA
<213>  Artificial Sequence

<220>
<223>  qRT-PCR primer

<400>  45
gaaagggcca aacagagaga                                                   20


<210>  46
<211>  20
<212>  DNA
<213>  Artificial Sequence

<220>
<223>  qRT-PCR primer

<400>  46
gtaaatcaca cggcgctctt                                                   20


<210>  47

<211>  18
<212>  DNA
<213>  Artificial Sequence

<220>
<223>  qRT-PCR primer

<400>  47
tgagagggcc aagcaaag                                                    18


<210>  48
<211>  20
<212>  DNA
<213>  Artificial Sequence

<220>
<223>  qRT-PCR primer

<400>  48
ataaatcaca cggcgctctt                                                  20


<210>  49
<211>  22
<212>  DNA
<213>  Artificial Sequence

<220>
<223>  qRT-PCR primer

<400>  49
tccacaaaca gaacaagatg ct                                               22


<210>  50
<211>  22
<212>  DNA
<213>  Artificial Sequence

<220>
<223>  qRT-PCR primer

<400>  50
ccattttcca ctttttctga gg                                               22


<210>  51
<211>  20
<212>  DNA
<213>  Artificial Sequence

<220>
<223>  qRT-PCR primer

<400>  51
tccactacat gacggagcag                                                  20


<210>  52
<211>  22
<212>  DNA
<213>  Artificial Sequence

<220>
<223>  qRT-PCR primer

<400>  52
ccatcttcag ttctgctaaa cg                                                22


<210>  53
<211>  19
<212>  DNA
<213>  Artificial Sequence

<220>
<223>  qRT-PCR primer

<400>  53
tgttcagttc caccccaca                                                    19


<210>  54
<211>  19
<212>  DNA
<213>  Artificial Sequence

<220>
<223>  qRT-PCR primer

<400>  54
tctccacgac acccttctg                                                    19


<210>  55
<211>  20
<212>  DNA
<213>  Artificial Sequence

<220>
<223>  qRT-PCR primer

<400>  55
gttctacggc aggctagtgg                                                   20


<210>  56
<211>  22
<212>  DNA
<213>  Artificial Sequence

<220>
<223>  qRT-PCR primer

<400>  56
acaatccaga acttcccaga ac                                                22


<210>  57
<211>  20
<212>  DNA
<213>  Artificial Sequence

<220>
<223>  qRT-PCR primer

<400>  57
tccgatgaag cttacgcaat                                                   20


<210>  58
<211>  23
<212>  DNA
<213>  Artificial Sequence

<220>
<223>  qRT-PCR primer

<400>  58
   tgtaaatgtt ccttttcctc tgc                                                23


<210>  59
<211>  25
<212>  DNA
<213>  Artificial Sequence

<220>
<223>  qRT-PCR primer

<400>  59
ccttgaagtc ctagagtcca ataaa                                             25


<210>  60
<211>  21
<212>  DNA
<213>  Artificial Sequence

<220>
<223>  qRT-PCR primer

<400>  60
ctatggcagt gcaaaaacct c                                                 21


<210>  61
<211>  20
<212>  DNA
<213>  Artificial Sequence

<220>
<223>  qRT-PCR primer

<400>  61
caggctgctg taacgatgaa                                                   20


<210>  62
<211>  20
<212>  DNA
<213>  Artificial Sequence

<220>
<223>  qRT-PCR primer

<400>  62
gctttggtga ggtttgatcc                                                   20

<210>  63
<211>  20
<212>  DNA
<213>  Artificial Sequence

<220>
<223>  qRT-PCR primer

<400>  63
cctccgaaac catgaacttt                                                    20


<210>  64
<211>  20
<212>  DNA
<213>  Artificial Sequence

<220>
<223>  qRT-PCR primer

<400>  64
atgattctgc cctcctcctt                                                    20

## Description

### FIELD OF THE INVENTION

The invention relates to the field of prostate cancer prognosis and, more in particular, to methods for predicting the outcome of a prostate cancer patient based on the expression levels of a gene and/or of a gene signature, as well as to methods for selecting a patient to be treated for advanced, recurrent or metastatic prostate cancer.

### BACKGROUND ART

Prostate cancer is a deadly disease with one of the highest incidence among cancer in men. 10-25% of patients fail to respond to first line therapies (surgery or radiotherapy) and this subset of patients present poor prognosis and high morbidity. Therefore, it is of key importance to identify and stratify patients in order to establish novel therapeutic initiatives that reduce the rate of recurrence.

Approaches to identify and stratify patients based on gene expression datasets that predict recurrence have proven successful in other types of cancer. Currently, transcriptomics-based biomarkers have been defined in prostate cancer. In particular:
- Prolaris (http://www.prolaris.com/) is based on the identification of a molecular signature of tumor growth;
- Oncotype DX (http://www.oncotypedx.com/) measures gene expression within 4 main pathways that account for cancer aggressiveness; and
- Decipher (http://deciphertest.com) is based on a gene expression signature associated to high risk of metastasis.

However, there is still a need for biomarkers with prognostic and predictive potential in order to define therapies for those patients who are at risk of developing an aggressive form of the disease.

### SUMMARY OF THE INVENTION

The present invention relates to the surprising finding that the expression levels of several genes involved in the cellular energy metabolism pathway are useful in prognosis of prostate cancer patients. This method can be used to determine those patients who are at a high risk of recurrence or metastasis. The method also allows for the identification of patients that can exhibit a better response to cytotoxic agents, improving financial and health outcomes.

Thus, in a first aspect, the invention relates to a method for determining the prognosis of a patient suffering from prostate cancer (hereinafter, first method of the invention) which comprises the determination in a sample from said patient of the expression levels of PGC 1A in a sample from the patient, wherein
a) an increase in the expression level of PGC 1A with respect to a reference value is indicative of a decreased risk of negative outcome of the patient; or
b) a decrease in the expression level of PGC1A with respect to a reference value is indicative of an increased risk of negative outcome of the patient.

In a second aspect, the invention also relates to a method for determining the prognosis of a patient suffering from prostate cancer which comprises the determination of the average expression levels of the PGC1A, ACACB, ANG, ATP5L, GSTM4, NDUFA4, NNT, PRKRA, RNASE4, and TP53INP2 genes in a sample from the patient, wherein
a) an increased average expression of said genes with respect to a reference value is indicative of a decreased risk of negative outcome for the patient; or
b) a decreased average expression of said genes with respect to a reference value is indicative of an increased risk of negative outcome of the patient.

In a third aspect, the invention relates to a method for predicting the response to therapy of a patient suffering from prostate cancer, which comprises the determination of the expression level of PGC 1A in a sample from the patient, wherein
a) an increase in the expression level of PGC1A with respect to a reference value is indicative of a positive response of the patient to therapy; or wherein
b) a decrease in the expression level of PGC1A with respect to a reference value is indicative of a negative response of the patient to therapy.

In a fourth aspect, the invention relates to a method for predicting the response to therapy of a patient suffering from prostate cancer, which comprises the determination of the average expression level of the PGC1A, ACACB, ANG, ATP5L, GSTM4, NDUFA4, NNT, PRKRA, RNASE4, and TP53INP2 genes in a sample from the patient, wherein
a) an increased average expression of said genes with respect to a reference value is indicative of a positive response of the patient to therapy; or
b) a decreased average expression of said genes with respect to a reference value is indicative of a negative response of the patient to therapy.

In a fifth aspect, the invention relates to a method for the identification of a patient to be treated for advanced, recurrent or metastatic prostate cancer which comprises the determination of the expression level of PGC 1A in a sample from the patient, wherein a decrease in the expression level of PGC1A with respect to a reference value is indicative that the patient is selected for treatment for advanced prostate cancer..

In a sixth aspect, the invention also relates to a method for selecting a patient to be treated for advanced, recurrent or metastatic prostate cancer which comprises the determination of the average expression levels of PGC1A, ACACB, ANG, ATP5L, GSTM4, NDUFA4, NNT, PRKRA, RNASE4, and TP53INP2 genes in a sample from the patient, wherein a decreased average expression of said genes with respect to a reference value is indicative that the patient is selected for treatment for advanced prostate cancer.

In a seventh aspect, the invention relates to a method of treatment of advanced, recurrent or metastatic prostate cancer to a patient in need thereof which comprises the administration of a therapy to said patient, wherein the patient has been selected using a method which comprises the determination of the expression level of PGC1A in a sample from the patient, wherein a decrease in the expression level of PGC1A with respect to a reference value is indicative that the patient is selected for treatment for advanced prostate cancer.

In an eighth aspect, the invention also relates to a method of treatment of advanced, recurrent or metastatic prostate cancer to a patient in need thereof which comprises the administration of a therapy to said patient, wherein the patient has been selected using a method which comprises the determination of the average expression levels of the PGC1A, ACACB, ANG, ATP5L, GSTM4, NDUFA4, NNT, PRKRA, RNASE4, and TP53INP2 genes in a sample from the patient, wherein a decreased average expression of said genes with respect to a reference value is indicative that the patient is selected for treatment for advanced prostate cancer.

In a ninth aspect, the invention relates to a kit or assay device comprising reagents adequate for the determination of the expression levels of PGC1A, or for the determination of the average expression level of the PGC1A, ACACB, ANG, ATP5L, GSTM4, NDUFA4, NNT, PRKRA, RNASE4, and TP53INP2 genes, wherein said reagents comprise at least 10% of the reagents present in the kit.

Additionally, the invention relates to the use of
- a reagent specific for the determination of the expression levels of PGC1A, or
- a kit or assay device as described above
for predicting the outcome of a patient suffering from prostate cancer or for predicting the response to therapy of a patient suffering from prostate cancer.

### BRIEF DESCRIPTION OF THE DRAWINGS

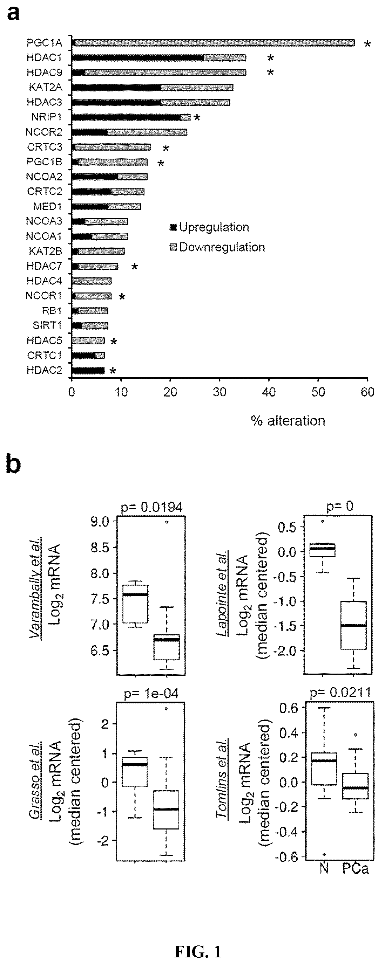

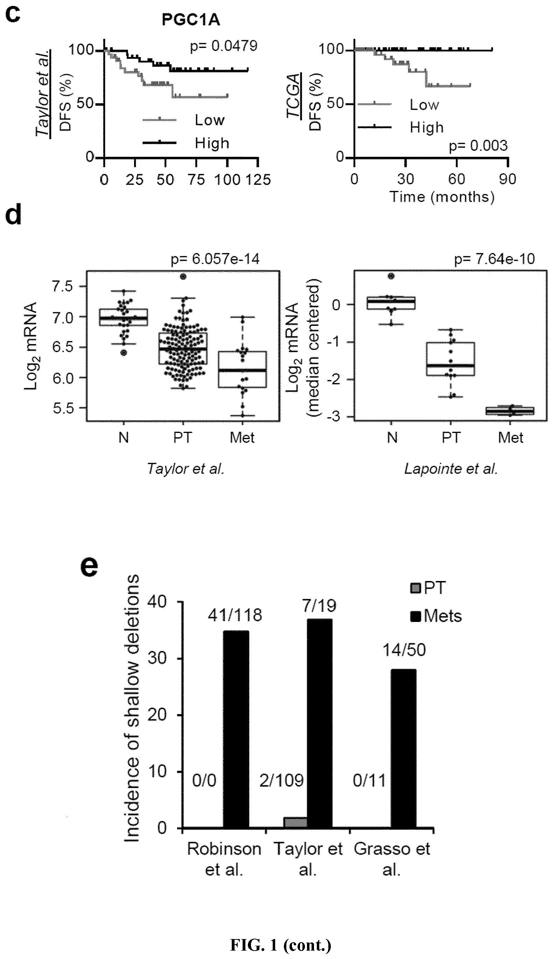

**FIG. 1** **shows that PGC1A is down-regulated in prostate cancer. a**, Frequency of alterations (differences greater than 2-fold vs. mean expression of non-tumor biopsies) in the expression of 23 master co-regulators of metabolism in a cohort of 150 PCa patients. *, statistically different expression (p<0.05) of the indicated gene in PCa (n=150) vs. normal (n=29) patient specimens (according to Supplementary Fig. 1A). **b**, Gene expression levels of PGC1A in up to four additional PCa datasets (N: normal; PCa: prostate cancer). Sample size: Tomlins (Normal=23; PCa=52); Grasso (Normal=12; PCa=76); Lapointe (Normal=9; PCa=17); Varambally (Normal=6; PCa=13). **c**, Association of PGC1A with disease-free survival (DFS) in two PCa datasets (Low: 1st quartile distribution; High: 4th quartile distribution) (Sample size: TCGA provisional data, primary tumors n=240; Taylor, primary tumors n=131). **d**, PGC1A expression in normal prostate (N), primary tumor (PT) and metastatic (Met) specimens in Taylor and Lapointe datasets. Sample size: Taylor N=29, PT=131 and Met=19; Lapointe N=9, PT=13 and Met=4. **e**, Incidence of PGC1A shallow deletions in three independent datasets. Points outlined by circles indicate statistical outliers (d). Error bars represent minimum and maximum values (b and d). p, p-value. Statistic test: two-tailed Student T test (a, b), Kaplan-Meier estimator (c, DFS) and ANOVA (d).

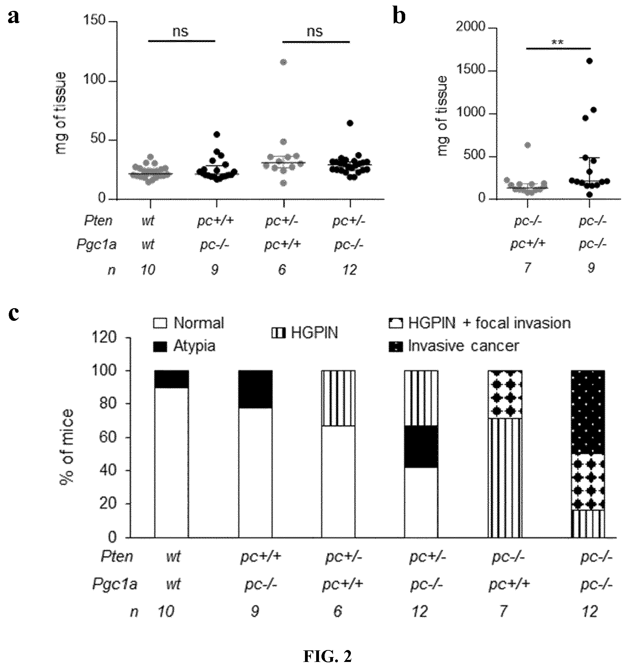

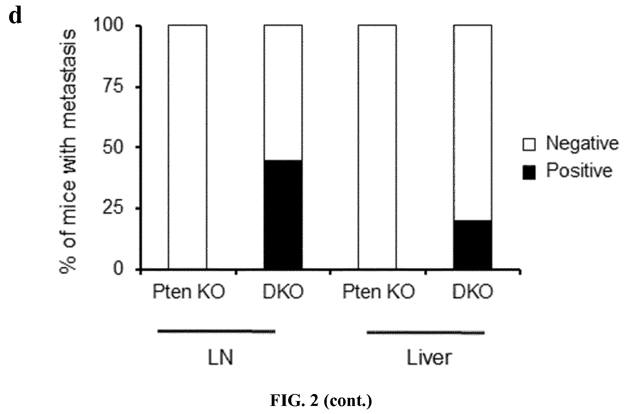

**FIG. 2** **shows that combined deletion of Pgc1a and Pten in the prostate epithelia results in prostate cancer progression and metastasis. a-b**, Comparison of anterior prostate lobe weights (when both anterior lobes were analyzed, the average was calculated and represented) between genotypes (Pten^{wt} Pgc1a^{wt} n=10 mice; Pten^{pc+/+} Pgc1a^{pc-/-} n=9 mice; Pten^{pc+/-} Pgc1a^{pc+/+} n=6 mice; Pten^{pc+/-} Pgc1a^{pc-/-} n=12 mice; Pten^{pc-/-} Pgc1a^{pc+/+} n=7 mice; Pten^{pc-/-} Pgc1a^{pc-/-} n=9 mice; pc, prostate-specific allelic changes; +, Wildtype allele; -, deleted allele; wt: any given genotype resulting in the lack of deletion of Pgc1a or Pten alleles). **c**, Histopathological characterization of the prostate (HGPIN: High-grade prostatic intraepithelial neoplasia) in the indicated genotypes (Pten^{wt} Pgc1a^{wt} n=10 mice; Pten^{pc-/-} Pgc1a^{pc-/-} n=9 mice; Pten^{pc+/-} Pgc1a^{pc+/+} n=6 mice; Pten^{pc-/-} Pgc1a^{pc-/-} n=12 mice; Pten^{pc-/-} Pgc1a^{pc+/+} n=7 mice; Pten^{pc-/-} Pgc1a^{pc-/-} n=12 mice; pc, prostate-specific allelic changes; +, Wildtype allele; -, deleted allele; wt: any given genotype resulting in the lack of deletion of Pgc1a or Pten alleles). **d**, Quantification of the frequency of metastatic lesions in lymph nodes (LN) and liver of Pten KO (5 mice) and DKO (9 mice) mice. DKO; Pten^{pc-/-}, Pgc1a^{pc-/-}. n.s.: not significant; **p<0.01. Error bars indicate median with interquartile range (a, b). Statistic test: two-tailed Mann Whitney U test (a, b).

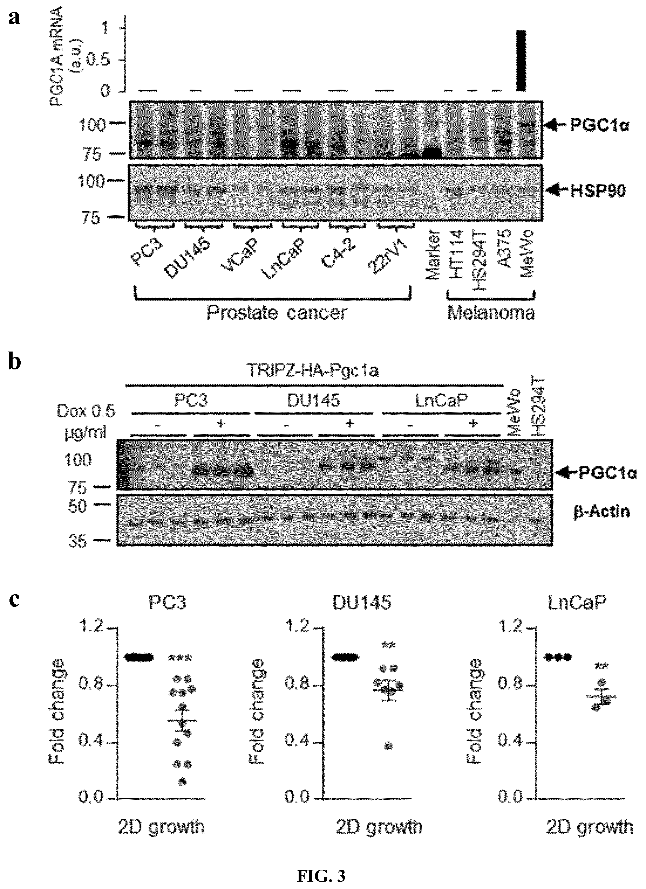

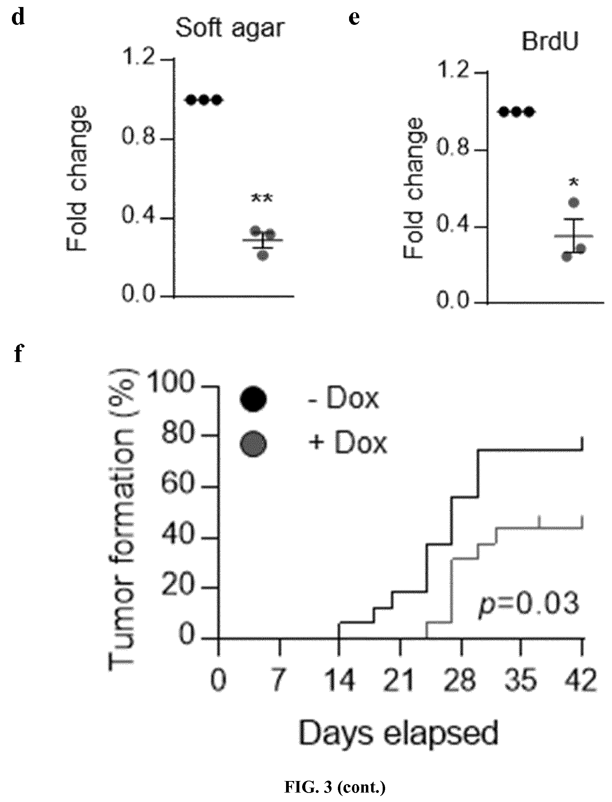

**FIG. 3** **shows that PGC1A inhibits growth in prostate cancer cell lines. a**, Analysis of Pgc1a expression by qRT-PCR (top histogram) and western blot in a panel of prostate cancer cell lines (technical duplicates are shown), using melanoma cell lines as positive (MeWo) and negative (HT114, HS294T and A375) controls (n=3, independent experiments). **b**, Representative experiment of Pgc1a expression in PC3, DU145 and LnCaP cell lines after treatment with 0.5 µg/ml doxycycline (Dox) (similar results were obtained in three independent experiments). **c**, Relative cell number quantification in Pgc1a expressing and non-expressing cells. Data is represented as cell number at day 6 relative to - Dox cells (n=12 in PC3; n=7 in DU145; n=3 in LnCaP, independent experiments). **d-e**, Effect of Pgc1a expression on anchorage-independent growth (d; n=3, independent experiments) and BrdU incorporation (e; n=3, independent experiments) in PC3 cells. **f**, Evaluation of tumour formation in xenotransplantation experiments (n=14 injections; 2 injections per mouse). Error bars represent standard deviation of the mean (c), s.e.m. (d, e). Statistic tests: two-tailed Student T test (c, d and e), log-rank test (f,). p, p-value. *p < 0.05, **p < 0.01, ***p < 0.001.

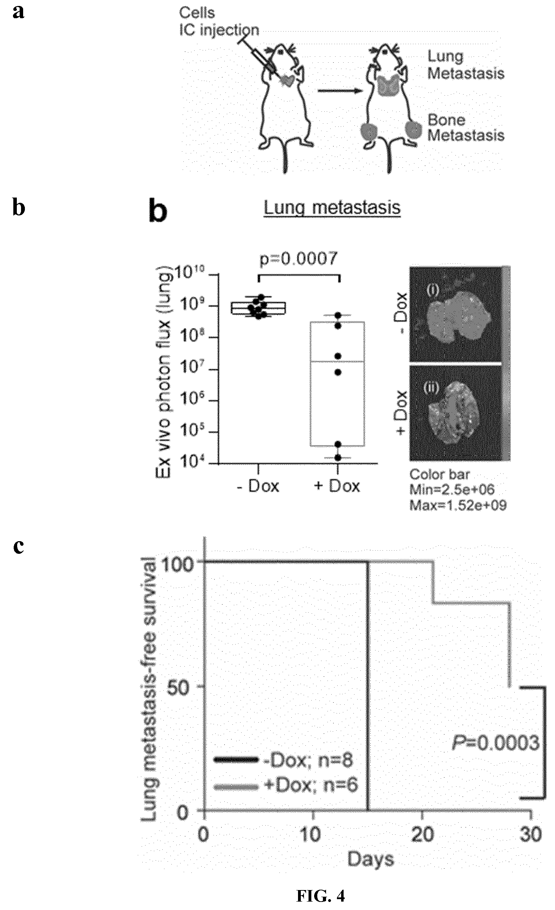

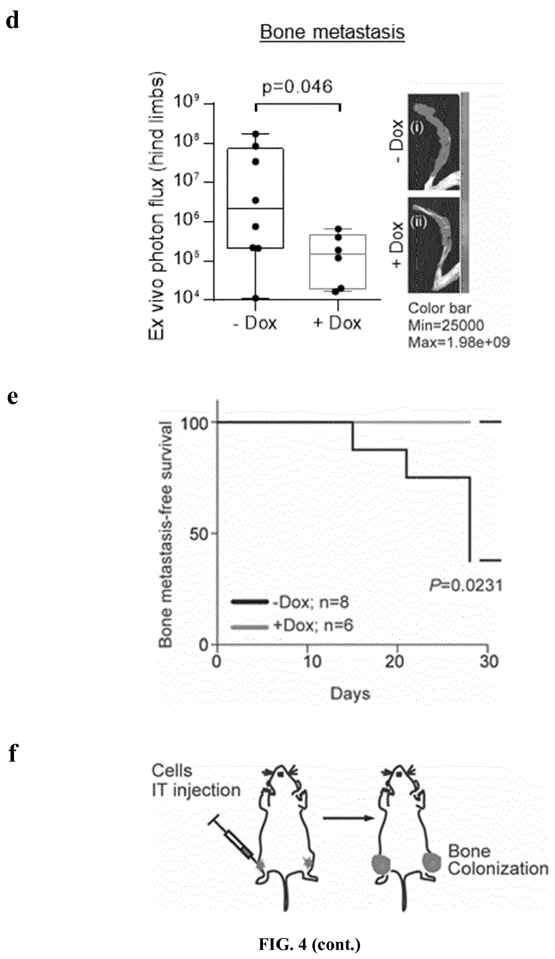

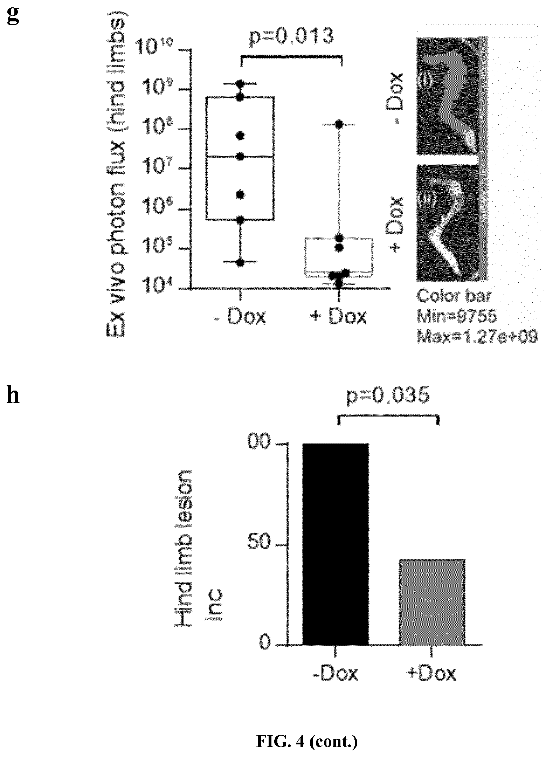

**FIG. 4** **shows that PGC1A inhibits metastasis in prostate cancer cell-derived xenografts. a**, Schematic representation of metastasis assay through intra-cardiac injection. **b-e**, Evaluation of metastatic capacity of Pgc1a-expressing PC3 cells using intra-cardiac xenotransplant assays (n=8 mice for - Dox and n=6 mice for + Dox). Luciferase-dependent signal intensity (b, d) and metastasis-free survival curves (c, e) of PCa cells in lungs (b, c) and limbs (d, e) was monitored for up to 28 days. Representative luciferase images are presented referred to the quantification plots. In hind limb photon flux analysis, average signal from two limbs per mouse is presented. (i) and (ii) depict tibia photon flux images from specimens that are proximal to the median signal in - Dox and + Dox, respectively. **f**, Schematic representation of bone metastasis assay through intra-tibial injection. **g-h**, Evaluation of the metastatic capacity of Pgc1a-expressing PC3 cells using intra-tibial xenotransplant assays (n=7 mice) Photon flux quantification at 20 days (g) and incidence of metastatic lesions at the end point (h). Representative luciferase images are presented referred to the quantification plots. For photon flux analysis, average signal from two limbs per mouse is presented. For incidence analysis, mice with at least one limb yielding luciferase signal > 50.000 units were considered metastasis-positive. (i) and (ii) depict tibia photon flux images from specimens that are proximal to the median signal in - Dox and + Dox, respectively. + Dox: Pgc1a induced conditions; - Dox: Pgc1a non-expressing conditions. Error bars represent median with interquartile range. Statistic tests: one-tailed Mann-Whitney U test b, d, g), log-rank test (c, e) and Fisher's exact test (h). p, p-value. *p < 0.05, **p < 0.01, ***p< 0.001.

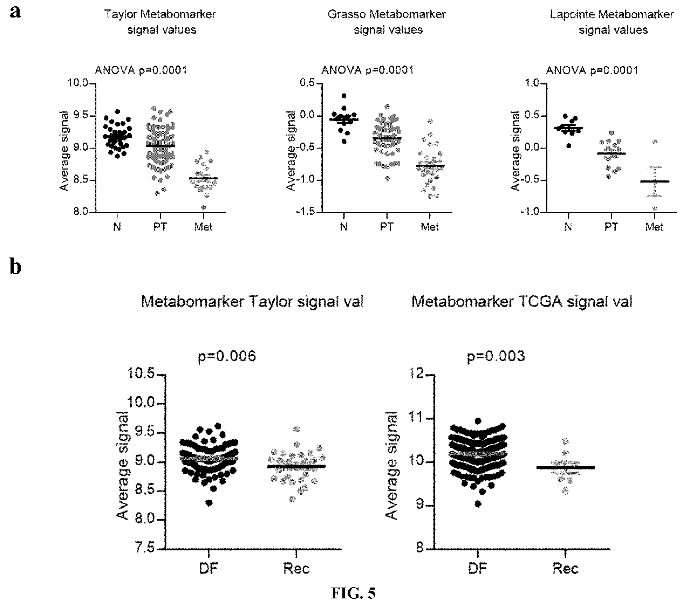

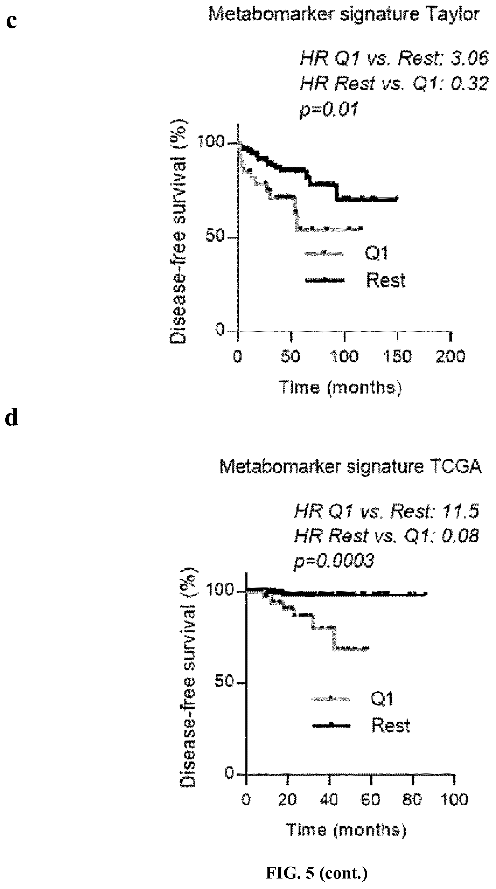

**FIG. 5** **shows that a 10-gene PGC1a signaling-based metabomarker gene signature exhibits prognostic potential. a**, Average signal of the gene signature per patient in specimens without pathology (N), with primary tumors (PT) or with metastasis (Met) in Taylor et al, Grasso et al datasets and Lapointe et al datasets. Schematic representation of metastasis assay through intra-cardiac injection. **b**, Average signal of the gene signature per patient in primary tumor patients who later develop (Rec) or not (DF) recurrence in Taylor et al and TCGA datasets. **c**, **d**, disease-free survival of patients exhibiting low average signal of the gene signature (first quartile, lower 25%, Q1) versus patients with high average signal of the gene signature (Rest, quartiles 2-4) in the primary tumor tissue, using Taylor et al., and TCGA datasets.

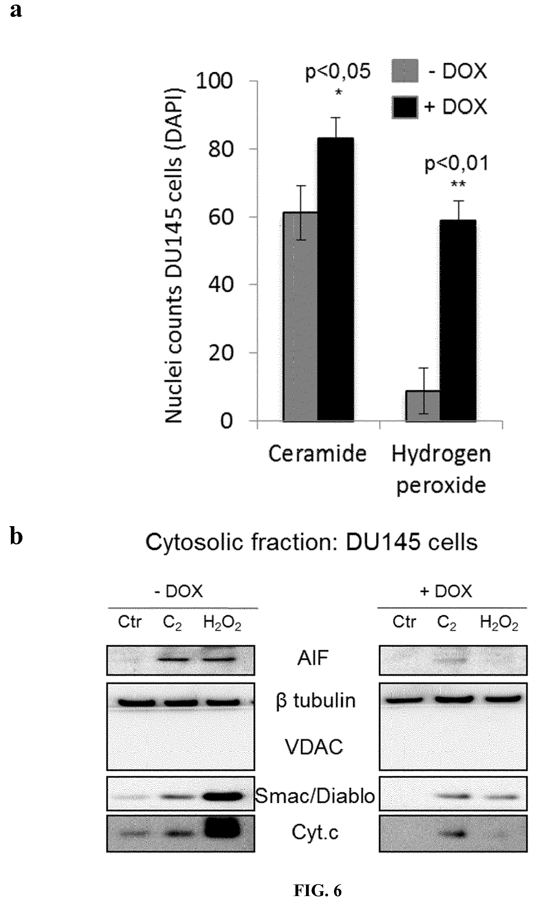

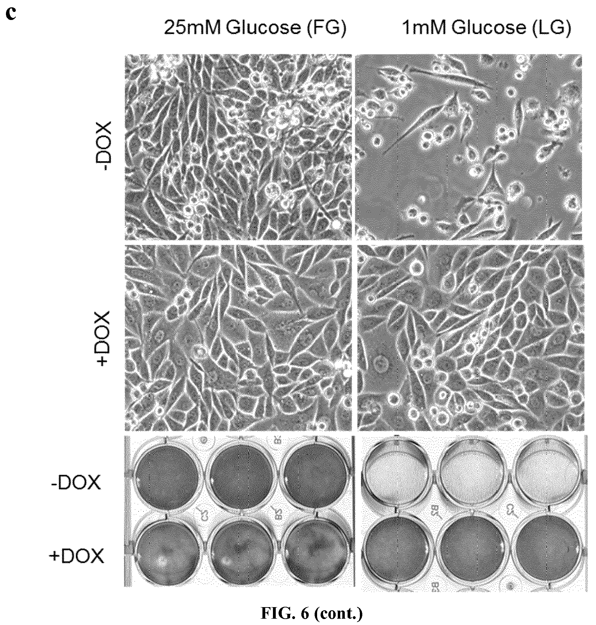

**FIG. 6** **shows that the absence of PGC1A increases the sensitivity of prostate cancer cells to cytotoxic insults. a**, cell number upon c2-ceramide or hydrogen peroxide treatment in DU145 prostate cancer cells expressing (+ Dox) or not expressing (- Dox) PGC1A. **b**, apoptotic markers in cells and experimental conditions showing increased apoptotic cell death in DU145 cells not expressing PGC1A after the indicated cytotoxic stimuli. **c**, micrographs (top panels) or crystal violet (readout of cell number, plates in lower panels) presenting the response of PGC1A expressing (+ Dox) and not expressing (- Dox) PC3 prostate cancer cells to glucose shortage. Increased cell death and reduced cell number is shown in the absence of PGC1A.

### DETAILED DESCRIPTION OF THE INVENTION

### Methods for predicting the outcome of a patient suffering from prostate cancer

The inventors have identified that the expression levels of a gene and/or of a gene signature involved in the cellular energy metabolism pathway under the control of PGC1A are reliable markers for predicting the outcome of a patient suffering from prostate cancer.

Therefore, in a first aspect, the invention relates to a method for determining the prognosis of a patient suffering from prostate cancer which comprises the determination in a sample from said patient of the expression levels of PGC1A in a sample from the patient (hereinafter, first method of the invention), wherein
a) an increase in the expression level of PGC1A with respect to a reference value is indicative of a decreased risk of negative outcome of the patient; or
b) a decrease in the expression level of PGC1A with respect to a reference value is indicative of an increased risk of negative outcome of the patient.

The first method of the invention comprises comparing the expression level of the PGC1A gene with a reference value.

"Reference value", as used herein in the context of the first method of the invention, refers to a laboratory value used as a reference for values/data obtained by laboratory examinations of subjects or samples collected from subjects. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

The reference value for the expression level ofPGC1A is the mean level of expression of PGC1A in a pool of samples from primary prostate tumors.

In a preferred embodiment, the reference value is the expression levels of the gene of interest in a pool obtained from primary prostate tumor tissues obtained from patients. This pool will include patients with good prognosis and patients with bad prognosis and therefore, the expression levels would be an average value of the values found in the different types of patients.

In another embodiment, the reference value is the expression levels of the gene of interest in primary prostate tumor tissue obtained from a patient or patients identified as patients having a good prognosis. In another embodiment, the reference value is the expression levels of the gene of interest in a tumor tissue obtained from a patient or patients identified as patients having a bad prognosis.

In case that the reference value is the expression level of the gene of interest obtained from primary prostate tumor tissues from patients having a good prognosis, then patients can be identified as having poor prognosis if the expression levels are lower than the reference value. In case that the reference value is the expression level of the gene of interest obtained from tumor tissues from patients having a poor prognosis, then patients can be identified as having good prognosis if the expression levels are higher than the reference value.

The sample collection from which the reference level is derived will preferably be formed by subjects suffering from the same type of cancer as the patient object of the study. Moreover, a reference value has to be established for each gene to be measured. In another embodiment, the quantity of any one or more biomarkers in a sample from a tested subject may be determined directly relative to the reference value (e.g., in terms of increase or decrease, or fold-increase or fold-decrease). Advantageously, this may allow to compare the quantity of any one or more biomarkers in the sample from the subject with the reference value (in other words to measure the relative quantity of any one or more biomarkers in the sample from the subject vis-a-vis the reference value) without the need to first determine the respective absolute quantities of said one or more biomarkers.

Once this reference value is established, the level of this marker expressed in tumor tissues from subjects can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

The comparison of the expression levels of the gene of interest with the reference value allows determining whether the patient will show a good or poor prognosis.

In the first method of the invention, an increase in the expression level of PGC1A with respect to a reference value is indicative of a decreased risk of negative outcome of the patient, whereas a decrease in the expression level of PGC1A with respect to a reference value is indicative of an increased risk of negative outcome of the patient.

As used herein, "a decreased risk of negative outcome" indicates that the subject is expected (e.g. predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for a heterogeneous cancer patient population. In the study of Paik et al. (2004), an overall "low" risk of recurrence was considered to be lower than 15 percent. The risk will also vary in function of the time period. The time period can be, for example, five years, ten years, fifteen years or even twenty years after initial diagnosis of cancer or after the prognosis is made.

As used herein, "an increased risk of negative outcome" indicates that the subject is expected, i.e. predicted, to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. The risk will also vary in function of the time period. The time period can be, for example, five years, ten years, fifteen years or even twenty years of initial diagnosis of cancer or after the prognosis was made.

In a second aspect, the invention also relates to a method for determining the prognosis of a patient suffering from prostate cancer which comprises the determination of the average expression levels of the PGC1A, ACACB, ANG, ATP5L, GSTM4, NDUFA4, NNT, PRKRA, RNASE4, and TP53INP2 genes in a sample from the patient (hereinafter, second method of the invention), wherein
a) an increased average expression of said genes with respect to a reference value is indicative of a decreased risk of negative outcome for the patient; or
b) an decreased average expression of said genes with respect to a reference value is indicative of an increased risk of negative outcome of the patient.

The second method of the invention comprises comparing the average expression level of the PGC1A, ACACB, ANG, ATP5L, GSTM4, NDUFA4, NNT, PRKRA, RNASE4, and TP53INP2 genes with a reference value.

"Reference value", as used herein in the context of the second method of the invention, refers to a laboratory value used as a reference for values/data obtained by laboratory examinations of subjects or samples collected from subjects. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

The reference value for the average expression level of the PGC1A, ACACB, ANG, ATP5L, GSTM4, NDUFA4, NNT, PRKRA, RNASE4, and TP53INP2 genes is the mean expression level of said genes in a pool of samples from primary prostate tumors.

In a preferred embodiment, the "average expression" of the PGC1A, ACACB, ANG, ATP5L, GSTM4, NDUFA4, NNT, PRKRA, RNASE4, and TP53INP2 genes may be obtained by first determining the individual expression level for each gene and then calculating the average of the individual expression levels for all genes. In a different embodiment, the "average expression" of the genes may be directly obtained by determining the expression level for all genes. In this embodiment, individual expression levels for each gene are not calculated. Since all genes have the same directionality in their altered condition (i.e. downregulation), the average signal informs on the activity of the cellular energy metabolism pathway.

In a preferred embodiment, the reference value is the expression levels of the genes of interest in a pool obtained from primary prostate tumor tissues obtained from patients. This pool will include patients with good prognosis and patients with bad prognosis and therefore, the expression levels would be an average value of the values found in the different types of patients.

In another embodiment, the reference value is the expression levels of the genes of interest in primary prostate tumor tissue obtained from a patient or patients identified as patients having a good prognosis. In another embodiment, the reference value is the expression levels of the genes of interest in a tumor tissue obtained from a patient or patients identified as patients having a bad prognosis.

In case that the reference value is the expression level of the genes of interest obtained from primary prostate tumor tissues from patients having a good prognosis, then patients can be identified as having poor prognosis if the expression levels are lower than the reference value. In case that the reference value is the expression level of the genes of interest obtained from tumor tissues from patients having a poor prognosis, then patients can be identified as having good prognosis if the expression levels are higher than the reference value.

The sample collection from which the reference level is derived will preferably be formed by subjects suffering from the same type of cancer as the patient object of the study. Moreover, a reference value has to be established for each gene to be measured. In another embodiment, the quantity of any one or more biomarkers in a sample from a tested subject may be determined directly relative to the reference value (e.g., in terms of increase or decrease, or fold-increase or fold-decrease). Advantageously, this may allow to compare the quantity of any one or more biomarkers in the sample from the subject with the reference value (in other words to measure the relative quantity of any one or more biomarkers in the sample from the subject vis-a-vis the reference value) without the need to first determine the respective absolute quantities of said one or more biomarkers.

Once this reference value is established, the level of this marker expressed in tumor tissues from subjects can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

The comparison of the expression levels of the genes of interest with the reference value allows determining whether the patient will show a good or poor prognosis.

In the second method of the invention, an increase in the average expression level of the PGC1A, ACACB, ANG, ATP5L, GSTM4, NDUFA4, NNT, PRKRA, RNASE4, and TP53INP2 genes with respect to a reference value is indicative of a decreased risk of negative outcome of the patient, whereas a decrease in the average expression level of the PGC1A, ACACB, ANG, ATP5L, GSTM4, NDUFA4, NNT, PRKRA, RNASE4, and TP53INP2 genes with respect to a reference value is indicative of an increased risk of negative outcome of the patient.

As used herein, the set of genes PGC1A, ACACB, ANG, ATP5L, GSTM4, NDUFA4, NNT, PRKRA, RNASE4, and TP53INP2 is known as the metabomarker or metabomarker gene signature. As used herein, the term "gene signature" refers to a set of one or more differentially expressed genes that are statistically significant and characteristic of the biological differences between two or more cell samples, e.g., control subjects or prostate cancer patients with increased risk of negative outcome. A signature may be expressed as a number of individual unique probes complementary to signature genes whose expression is detected when a cRNA product is used in microarray analysis or in a PCR reaction. A signature may be exemplified by a particular set of genes making up a biomarker. The signature comprises the PGC1A, ACACB, ANG, ATP5L, GSTM4, NDUFA4, NNT, PRKRA, RNASE4, and TP53INP2 genes.

The second method of the invention involves determining the expression level of the genes forming the metabomarker gene signature as average expression level. As used herein, the expression "average expression level" is understood as arithmetic average of logarithm-transformed values of gene expression levels, in whatever units are chosen, as measured on any applicable platform, as listed above. The expression average should be understood in the wide sense as covering not only the arithmetic mean but also other calculation methods, such as the geometric mean.

As used herein, "a decreased risk of negative outcome" indicates that the subject is expected (e.g. predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for a heterogeneous cancer patient population. In the study of Paik et al. (2004), an overall "low" risk of recurrence was considered to be lower than 15 percent. The risk will also vary in function of the time period. The time period can be, for example, five years, ten years, fifteen years or even twenty years after initial diagnosis of cancer or after the prognosis is made.

As used herein, "an increased risk of negative outcome" indicates that the subject is expected, i.e. predicted, to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. The risk will also vary in function of the time period. The time period can be, for example, five years, ten years, fifteen years or even twenty years of initial diagnosis of cancer or after the prognosis was made.

The term "outcome" refers to a prediction of medical prognosis, for example, a poor or good outcome (e.g., likelihood of long-term survival, overall survival, disease-specific survival, progression-free survival or disease-free survival); a negative prognosis, or poor outcome, includes a prediction of relapse, disease progression (e.g., tumor growth or metastasis, or drug resistance), or mortality; whereas a positive prognosis, or good outcome, includes a prediction of disease remission, (e.g., disease-free status), amelioration (e.g., tumor regression), or stabilization.

Any parameter which is widely accepted for determining the outcome of a patient can be used in the present invention including, without limitation:
- overall survival rate, as used herewith, relates to the percentage of people in a study or treatment group who are alive for a certain period of time after they were diagnosed with or treated for a disease, such as cancer.
- disease-specific survival rate which is defined as the percentage of people in a study or treatment group who have not died from a specific disease in a defined period of time.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumor control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- time to progression (TTP), as used herein, relates to the time since a disease is treated until the disease starts to get worse.
- six-month progression free survival or "PFS6" rate which, as used herein, relates to the percentage of subjects who are free of progression in the first six months after the initiation of the therapy and
- median survival which, as used herein, relates to the time at which half of the subjects enrolled in the study are still alive.

The term "patient" or "subject" refers to a human male of any age or race. In a preferred embodiment, the subject has not been treated with chemotherapy or radiotherapy prior to the determination of the expression levels of the gene or genes of interest. In yet another embodiment, the patient has undergone surgical resection of the tumor.

As used herein, "sample" or "biological sample" means biological material isolated from a subject. The biological sample may contain any biological material suitable for determining the expression level of PGC1A. The sample can be isolated from any suitable biological tissue or fluid such as, for example, tumor tissue, blood, plasma, serum, sputum, bronchoalveolar lavage, urine or cerebral spinal fluid (CSF).

In a preferred embodiment, the sample contains tumor cells. In a more preferred embodiment, the sample containing tumor cells is a tumor tissue sample. The tumor tissue sample is understood as the tissue sample originating from the primary tumor or from a distant metastasis. Said sample can be obtained by conventional methods, for example biopsy, using methods well known by the person skilled in related medical techniques. Alternatively, the tumor tissue sample may be a sample of a tumor which has been surgically resected. The methods for obtaining a biopsy sample include splitting a tumor into large pieces, or microdissection, or other cell separating methods known in the art. The tumor cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing.

The term "expression level" of a gene as used herein refers to the measurable quantity of gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene.

The expression levels of the PGC1A gene can be determined by measuring the levels of mRNA encoded by said gene, or by measuring the levels of the protein encoded by said gene, i.e. PGC1A protein, or of variants thereof. The expression levels of the PGC1A, ACACB, ANG, ATP5L, GSTM4, NDUFA4, NNT, PRKRA, RNASE4, and TP53INP2 genes can be determined by measuring the levels of mRNA encoded by said genes, or by measuring the levels of the protein encoded by said genes, i.e. PGC1A, ACACB, ANG, ATP5L, GSTM4, NDUFA4, NNT, PRKRA, RNASE4, and TP53INP2 proteins, or of variants thereof.

In order to measure the mRNA levels of the PGC1A gene or of the PGC1A, ACACB, ANG, ATP5L, GSTM4, NDUFA4, NNT, PRKRA, RNASE4, and TP53INP2 genes, the biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor.

In a preferred embodiment samples can be obtained from fresh tumor tissue or from OCT embedded frozen tissue. In another preferred embodiment samples can be obtained by bronchoscopy and then paraffin-embedded.

Determination of the levels of mRNA of the PGC1A gene or of the PGC1A, ACACB, ANG, ATP5L, GSTM4, NDUFA4, NNT, PRKRA, RNASE4, and TP53INP2 genes can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the levels of the above genes can also be carried out by Fluorescence In Situ Hybridization, including variants such as Flow-FISH, qFiSH and double fusion FISH (D-FISH) as described in WO2010030818. The levels of the mRNA of the different genes can also be determined by nucleic acid sequence based amplification (NASBA) technology.

In a preferred embodiment, the gene mRNA expression levels are often determined by reverse transcription polymerase chain reaction (RT-PCR). The detection can be carried out in individual samples or in tissue microarrays.

Thus, in a particular embodiment, the mRNA expression levels of the PGC1A gene or of the PGC1A, ACACB, ANG, ATP5L, GSTM4, NDUFA4, NNT, PRKRA, RNASE4, and TP53INP2 genes are determined by quantitative PCR, preferably, Real-Time PCR. The detection can be carried out in individual samples or in tissue microarrays.

Alternatively, in another embodiment of the first and second methods of the invention, the expression level of the PGC1A gene or of the PGC1A, ACACB, ANG, ATP5L, GSTM4, NDUFA4, NNT, PRKRA, RNASE4, and TP53INP2 genes is determined by measuring the expression of the polypeptide or of variants thereof. In a preferred embodiment the expression level of the protein or of variants thereof is determined by Western blot, ELISA or by immunohistochemistry.

The expression levels of Pgc1a or of the proteins encoded by the PGC1A, ACACB, ANG, ATP5L, GSTM4, NDUFA4, NNT, PRKRA, RNASE4, and TP53INP2 genes can be quantified by means of conventional methods, for example, using antibodies with a capacity to specifically bind to the proteins encoded by said genes (or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' and F(ab')₂, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

Alternatively, in another particular embodiment, the levels of Pgc1a or of the proteins encoded by the PGC1A, ACACB, ANG, ATP5L, GSTM4, NDUFA4, NNT, PRKRA, RNASE4, and TP53INP2 genes or of the variants thereof are determined by Western blot. Western blot is based on the detection of proteins previously resolved by gel electrophoreses under denaturing conditions and immobilized on a membrane, generally nitrocellulose, by the incubation with an antibody specific and a developing system (e.g. chemoluminiscent).

The term "PGC1A", also known as PGC1α LEM6, PGC-1v, PGC1, PPARGC1A and PPARGC1, as used herein refers to a gene encoding for Peroxisome proliferator-activated receptor gamma coactivator 1-alpha. The human gene is shown in the Ensembl database under accession number ENSG00000109819.

The term "ACACB", also known as ACCB, ACC2 and HACC275, as used herein refers to a gene encoding for Acetyl-CoA carboxylase 2. The human gene is shown in the Ensembl database under accession number ENSG00000076555.

The term "ANG", also known as RAA1, HEL168, RNASE5, ALS9 and RNASE4, as used herein refers to a gene encoding for Angiogenin. The human gene is shown in the Ensembl database under accession number ENSG00000214274.

The term "ATP5L", also known as ATP5JG, as used herein refers to a gene encoding for ATP synthase subunit g, mitochondrial. The human gene is shown in the Ensembl database under accession number ENSG00000167283.

The term "GSTM4", also known as GSTM4-4 and GTM4, as used herein refers to a gene encoding for Glutathione S-transferase Mu 4. The human gene is shown in the Ensembl database under accession number ENSG00000168765.

The term "NDUFA4", also known as CI-MLRQ, MLRQ and CI-9k, as used herein refers to a gene encoding for NDUFA4, mitochondrial complex associated. The human gene is shown in the Ensembl database under accession number ENSG00000189043.

The term "NNT", also known as GCCD4, as used herein refers to a gene encoding for nicotinamide nucleotide transhydrogenase. The human gene is shown in the Ensembl database under accession number ENSG00000112992.

The term "PRKRA", also known as PACT, DYT16, RAX and HSD14, as used herein refers to a gene encoding for Protein kinase, interferon-inducible double stranded RNA dependent activator. The human gene is shown in the Ensembl database under accession number ENSG00000180228.

The term "RNASE4", also known as RAB1 and RNS4, as used herein refers to a gene encoding for ribonuclease, RNase A family, 4. The human gene is shown in the Ensembl database under accession number ENSG00000258818.

The term "TP53INP2", also known as FLJ21759, C20orf110, DKFZp43400827, FLJ23500, DOR, dJ1181N3.1, DKFZp434B2411 and PINH, as used herein refers to a gene encoding for tumor protein p53 inducible nuclear protein 2. The human gene is shown in the Ensembl database under accession number ENSG00000078804.

In a preferred embodiment, the first and second methods of the invention further comprise the determination of one or more clinical parameters which are also indicative of the prognosis of the cancer. Such indicators include the presence or levels of known cancer markers, or can be clinical or pathological indicators (for example, age, tumor size, tumor histology, differentiation grade, clinical stage, family history and the like).

The person skilled in the art will understand that the determination of the prognosis is not needed to be correct for all the subjects (i.e., for 100% of the subjects). Nevertheless, the term requires enabling the identification of a statistically significant part of the subjects (for example, a cohort in a cohort study). Whether a part is statistically significant can be determined in a simple manner by the person skilled in the art using various well known statistical evaluation tools, for example, the determination of confidence intervals, determination of p values, Student's T test, Mann-Whitney test, etc. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

All the terms and embodiments described elsewhere herein are equally applicable to the first and second aspects of the invention.

Method for predicting the response to therapy of a patient suffering from prostate cancer In a third aspect, the invention relates to a method for predicting the response to therapy of a patient suffering from prostate cancer (hereinafter, third method of the invention), which comprises the determination of the expression level of PGC1A in a sample from the patient, wherein
a) an increase in the expression level of PGC1A with respect to a reference value is indicative of a positive response of the patient to therapy; or wherein
b) a decrease in the expression level of PGC1A with respect to a reference value is indicative of a negative response of the patient to therapy.

The term "reference value" has been described in the context of the first method of the invention. In a preferred embodiment, the reference value for the expression level of PGC1A is the mean expression levels of PGC1A in a pool obtained from primary prostate tumor tissues. In another embodiment, the reference value is the expression levels of the gene of interest in a primary prostate tumor tissue obtained from a patient or patients identified as patients having a good prognosis. In another embodiment, the reference value is the expression levels of the gene of interest in a primary prostate tumor tissue obtained from a patient or patients identified as patients having a bad prognosis.

In a fourth aspect, the invention relates to a method for predicting the response to therapy of a patient suffering from prostate cancer (hereinafter, fourth method of the invention), which comprises the determination of the average expression level of the PGC1A, ACACB, ANG, ATP5L, GSTM4, NDUFA4, NNT, PRKRA, RNASE4, and TP53INP2 genes in a sample from the patient, wherein
a) an increased average expression of said genes with respect to a reference value is indicative of a positive response of the patient to therapy; or
b) a decreased average expression of said genes with respect to a reference value is indicative of a negative response of the patient to therapy.

In a preferred embodiment, the "average expression" of the PGC1A, ACACB, ANG, ATP5L, GSTM4, NDUFA4, NNT, PRKRA, RNASE4, and TP53INP2 genes may be obtained by first determining the individual expression level for each gene and then calculating the average of the individual expression levels for all genes. In a different embodiment, the "average expression" of the genes may be directly obtained by determining the expression level for all genes. In this embodiment, individual expression levels for each gene are not calculated. Since all genes have the same directionality in their altered condition (i.e. downregulation), the average signal informs on the activity of the cellular energy metabolism pathway.

The term "reference value" has been described in the context of the second method of the invention. In a preferred embodiment, the reference value for the average expression level of the PGC1A, ACACB, ANG, ATP5L, GSTM4, NDUFA4, NNT, PRKRA, RNASE4, and TP53INP2 genes is the mean expression levels of said genes in a pool obtained from primary prostate tumor tissues. In another embodiment, the reference value is the expression levels of the genes of interest in a primary prostate tumor tissue obtained from a patient or patients identified as patients having a good prognosis. In another embodiment, the reference value is the expression levels of the genes of interest in a primary prostate tumor tissue obtained from a patient or patients identified as patients having a bad prognosis.

Once the reference value is established, the level of this marker expressed in tumor tissues from subjects can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level
The expression "positive response" when referred to the treatment for advanced, recurrent or metastatic prostate cancer, as used herein, refers to any response which is substantially better than that obtained with a saline control or placebo. The response can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition of metastasis; (6) enhancement of anti-tumor immune response, possibly resulting in regression or rejection of the tumor; (7) relief, to some extent, of one or more symptoms associated with the tumor; (8) increase in the length of survival following treatment; and/or (9) decreased mortality at a given point of time following treatment.

Positive clinical response may also be expressed in terms of various measures of clinical outcome as defined elsewhere herein. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

As used herein the term "a negative response" when referred to the treatment for advanced, recurrent or metastatic prostate cancer means that the treatment provides no reduction of the assessed symptoms of the cancer or causes an increase in the symptoms of the cancer.

All the terms and embodiments described elsewhere herein are equally applicable to the third and fourth aspects of the invention.

### Method for selecting a patient to be treated for advanced, recurrent or metastatic prostate cancer

In a fifth aspect, the invention relates to a method for the identification of a patient to be treated for advanced, recurrent or metastatic prostate cancer which comprises the determination of the expression level of PGC1A in a sample from the patient (hereinafter, fifth method of the invention), wherein a decrease in the expression level of PGC1A with respect to a reference value is indicative that the patient is selected for treatment for advanced prostate cancer. In a preferred embodiment, the reference value for the expression level of PGC1A is the mean level of expression of PGC1A in a pool of samples from primary prostate tumors.

In a sixth aspect, the invention also relates to a method for selecting a patient to be treated for advanced, recurrent or metastatic prostate cancer which comprises the determination of the average expression levels of the PGC1A, ACACB, ANG, ATP5L, GSTM4, NDUFA4, NNT, PRKRA, RNASE4, and TP53INP2 genes in a sample from the patient (hereinafter, sixth method of the invention), wherein a decreased average expression of said genes with respect to a reference value is indicative that the patient is selected for treatment for advanced prostate cancer. In a preferred embodiment, the reference value for the average expression levels of the PGC1A, ACACB, ANG, ATP5L, GSTM4, NDUFA4, NNT, PRKRA, RNASE4, and TP53INP2 genes is the mean expression level in a pool of samples from primary prostate tumors.

In a preferred embodiment, the sample from the patient is a sample containing tumor cells. In a more preferred embodiment, the sample containing tumor cells is tumor tissue. The tumor tissue may be a tumor biopsy or a surgically resected tumor.

In a preferred embodiment, the patient is a patient in which a primary prostate tumor has been surgically excised. In another preferred embodiment, the patient has not been treated with adjuvant therapy, such as chemotherapy or radiotherapy prior to the determination of the expression levels of the gene or genes of interest.

In prostate cancer therapy, a variety of treatments can be used in an attempt to eliminate or contain the cancer. Treatment for prostate cancer may involve active surveillance, hormonal therapy, radiation therapy including brachytherapy (prostate brachytherapy) and external beam radiation, High Intensity Focused Ultrasound (HIFU), chemotherapy, or some combination. Which option is best depends on the stage of the disease, the Gleason score, and the PSA level. Other important factors are the man's age, his general health, and his feelings about potential treatments and their possible side effects. Because all treatments can have significant side effects, such as erectile dysfunction and urinary incontinence, treatment discussions often focus on balancing the goals of therapy with the risks of lifestyle alterations.

Briefly, depending on the above mentioned factors (e.g., age of the subject, as well as the size, location and cancer phase), (i) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and antitumor agents; and/or (ii) immunotherapy, wherein the administered compound stimulates, enhances or strengthens the natural function of the immune system against cancer to recognize and eliminate the cancerous cells from the body, can be used. Thus, the method of the invention allows determining the response of the subject having prostate cancer to any treatment, particularly, to any cytotoxic and/or cytostatic treatment and, more specifically, to a treatment by means of chemotherapy, radiotherapy, antitumor agents or combinations thereof.

Suitable chemotherapy agents include but are not limited to alkylating agents [e.g., Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, Uramustine, etc.]; anti-metabolites [e.g., purine (azathioprine, mercaptopurine), pyrimidine (Capecitabine, Cytarabine, Fluorouracil, Gemcitabine), folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., Gleevec(TM)]; an epidermal growth factor receptor inhibitor [e.g., Iressa(TM), erlotinib (Tarceva(TM)), gefitinib, etc.]; an FPTase inhibitor [e.g., FTIs (R1 15777, SCH66336, L- 778, 123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltirexed (ZD 1694, Tomudex), ZD9331, 5-FU, etc.]; an S-adenosylmethionine decarboxylase inhibitor [e.g., SAM468A, etc.]; a DNA methylating agent [e.g., TMZ, etc.]; a DNA binding agent [e.g., PZA, etc.]; an agent which binds and inactivates 06-alkylguanine AGT [e.g., BG]; a c-ra/-1 antisense oligo-deoxynucleotide [e.g., ISIS-5132 (CGP- 69846A)]; tumor immunotherapy; a steroidal and/or nonsteroidal antiinflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Suitable chemotherapy agents are described with more detail in the literature, such as in The Merck Index in CD-ROM, 13th edition.

The term "adjuvant chemotherapy" as used herein means treatment of cancer with standard chemotherapeutic agents after surgery where all detectable disease has been removed, but where there still remains a risk of small amounts of remaining cancer. Adjuvant therapy can include chemotherapy or radiotherapy.

The term "chemotherapy" refers to the use of drugs to destroy cancer cells. The drugs are generally administered through oral or intravenous route. Sometimes, chemotherapy is used together with radiation treatment.

The term "radiotherapy" or "radiotherapeutic treatment" is a term commonly used in the art to refer to multiple types of radiation therapy including internal and external radiation therapies or radio immunotherapy, and the use of various types of radiations including X-rays, gamma rays, alpha particles, beta particles, photons, electrons, neutrons, radioisotopes, and other forms of ionizing radiations.

The determination of the expression levels of the gene or genes may be carried out by determining the levels of the corresponding mRNAs or by determining the levels of the polypeptides encoded by said genes, as described earlier.

In a preferred embodiment, the sample is a tumor tissue sample, which may be either a biopsy of the tumor or a fragment of the tumor obtained after the tumor has been surgically resected.

In a preferred embodiment, determination of the expression levels of the gene or genes of interest is carried out by determination of the levels of the corresponding mRNAs. In another embodiment, determination of the expression levels of the gene or genes of interest is carried out by determination of the levels of the corresponding polypeptides encoded by said genes.

In a preferred embodiment, the fifth and sixth methods of the invention further comprise the determination of one or more clinical parameters which are also indicative of the prognosis of prostate cancer. Such indicators include the presence or levels of known cancer markers, or can be clinical or pathological indicators (for example, age, tumor size, tumor histology, differentiation grade, clinical stage, family history and the like).

The fifth and sixth methods of the invention comprise correlating the expression levels of the genes with a reference value. The term "reference value" has been described in the context of the first and second methods of the invention. In a preferred embodiment, the reference value is the mean expression levels of the gene or genes of interest in a pool obtained from primary prostate tumor tissues. In another embodiment, the reference value is the mean expression levels of the gene or genes of interest in a primary prostate tumor tissue obtained from a patient or patients identified as patients having a good prognosis. In another embodiment, the reference value is the mean expression levels of the gene or genes of interest in a primary prostate tumor tissue obtained from a patient or patients identified as patients having a bad prognosis.

Once this reference value is established, the level of this marker expressed in tumor tissues from subjects can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level

The term "treatment", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

When the cancer has metastasized, systemic treatments including but not limited to chemotherapy, hormone treatment, immunotherapy, or a combination thereof are used. Additionally, radiotherapy and/or surgery can be used. The choice of treatment generally depends on the type of primary cancer, the size, the location of the metastasis, the age, the general health of the patient and the types of treatments used previously.

All the terms and embodiments described elsewhere herein are equally applicable to these aspects of the invention.

### A method of treatment of advanced, recurrent or metastatic prostate cancer to a patient in need thereof

In a seventh aspect, the invention relates to a method of treatment of advanced, recurrent or metastatic prostate cancer to a patient in need thereof which comprises the administration of a therapy to said patient, wherein the patient has been selected using a method which comprises the determination of the expression level of PGC1A in a sample from the patient, wherein a decrease in the expression level of PGC1A with respect to a reference value is indicative that the patient is selected for treatment for advanced prostate cancer.

In an eighth aspect, the invention also relates to a method of treatment of advanced, recurrent or metastatic prostate cancer to a patient in need thereof which comprises the administration of a therapy to said patient, wherein the patient has been selected using a method which comprises the determination of the average expression level of the PGC1A, ACACB, ANG, ATP5L, GSTM4, NDUFA4, NNT, PRKRA, RNASE4, and TP53INP2 genes in a sample from the patient, wherein a decreased average expression of said genes with respect to a reference value is indicative that the patient is selected for treatment for advanced prostate cancer.

Appropriate method steps to select a patient in need of treatment of advanced, recurrent or metastatic prostate cancer have been described elsewhere herein. Suitable methods of treatment of advanced, recurrent or metastatic prostate cancer have also been described elsewhere herein.

All the terms and embodiments described elsewhere herein are equally applicable to these aspects of the invention.

### Personalized therapy to treat a patient in need thereof for advanced, recurrent or metastatic prostate cancer

The present invention also refers to suitable methods for their use in the treatment of advanced, recurrent or metastatic prostate cancer in a patient, wherein the patient is characterized by having a decreased expression of the PGC1A gene, or wherein the patient is characterized by having a decreased average expression of the PGC1A, ACACB, ANG, ATP5L, GSTM4, NDUFA4, NNT, PRKRA, RNASE4, and TP53INP2 genes.

Further, the present invention also refers to suitable methods for their use in the treatment of advanced, recurrent or metastatic prostate cancer in a patient, wherein the patient has been selected because said patient presents a decreased expression of the PGC1A gene, or wherein the patient has been selected because said patient presents a decreased average expression of the PGC1A, ACACB, ANG, ATP5L, GSTM4, NDUFA4, NNT, PRKRA, RNASE4, and TP53INP2 genes.

Additionally, the present invention also refers to suitable methods for their use in the treatment of advanced, recurrent or metastatic prostate cancer in a patient, wherein before the treatment the patient is selected by means of a method that comprises determining the expression level of the PGC1A gene, or wherein before the treatment the patient is selected by means of a method that comprises determining the average expression level of the PGC1A, ACACB, ANG, ATP5L, GSTM4, NDUFA4, NNT, PRKRA, RNASE4, and TP53INP2 genes.

Suitable methods of treatment of advanced, recurrent or metastatic prostate cancer have been described elsewhere herein.

All the terms and embodiments described elsewhere herein are equally applicable to these aspects of the invention.

### Kits and assay devices

In a final aspect, the invention relates to a kit or assay device comprising reagents adequate for the determination of the expression levels of PGC1A, or for the determination of the average expression level of the PGC1A, ACACB, ANG, ATP5L, GSTM4, NDUFA4, NNT, PRKRA, RNASE4, and TP53INP2 genes, wherein said reagents comprise at least 10% of the reagents present in the kit.

In the context of the present invention, "kit" or "assay device" is understood as a product or device containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

The reagents for use in the first or second methods of the invention may be formulated as a "kit" and thus, may be combined with one or more other types of elements or components (e.g., other types of biochemical reagents, containers, packages such as packaging intended for commercial sale, substrates to which the reagents are attached, electronic hardware components, etc.).

In a preferred embodiment, the reagents adequate for the determination of the expression levels of one or more genes comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit. Thus, in the particular case of kits comprising reagents for the determination of the average expression levels of the PGC1A, ACACB, ANG, ATP5L, GSTM4, NDUFA4, NNT, PRKRA, RNASE4, and TP53INP2 genes, the reagents specific for said gene (i.e. probes which are capable of hybridizing under stringent conditions to the PGC1A, ACACB, ANG, ATP5L, GSTM4, NDUFA4, NNT, PRKRA, RNASE4, and TP53INP2 genes) comprise at least 10%, at least 20%, at least 30%, at least 40%, at least 50%, at least 60%, at least 70%, at least 80%, at least 90% or at least 100% of the probes present in the kit. In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes.

In a preferred embodiment, the first component of the kit of the invention comprises probes which can specifically hybridize to the genes mentioned above.

The term "specifically hybridizing", as used herein, refers to conditions which allow hybridizing of two polynucleotides under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and the hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50° C.; (2) employ during hybridization a denaturing agent, such as formamide, for example, 50% (v/v) formamide with 0.1% bovine serum albumin/0.1 % Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mMNaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like. In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular subsequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogs thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA. Each probe is assayed in parallel with a probe with a changed base, preferably in a central position of the probe. The array is put into contact with a sample containing nucleic acids with sequences complementary to the probes of the array and the signal of hybridization with each of the probes and with the corresponding hybridization controls is determined. Those probes in which a higher difference is observed between the signal of hybridization with the probe and its hybridization control are selected. The optimization process can include a second round of optimization in which the hybridization array is hybridized with a sample that does not contain sequences complementary to the probes of the array. After the second round of selection, those probes having signals of hybridization lower than a threshold level will be selected. Thus, probes which pass both controls, i.e., which show a minimum level of unspecific hybridization and a maximum level of specific hybridization with the target nucleic acid are selected.

The microarrays of the invention contain not only specific probes for the polynucleotides indicating a determined pathophysiological situation, but also containing a series of control probes, which can be of three types: normalization controls, expression level controls and hybridization controls.

Probes suitable for use as expression controls correspond to genes expressed constitutively, such as genes encoding proteins which exert essential cell functions such as β-2-microglobulin, ubiquitin, ribosomal protein 18S, cyclophilin A, transferrin receptor, actin, GAPDH, tyrosine 3-monooxygenase/tryptophan 5-monooxygenase activation protein (YWHAZ), HPRT, and IPO8.

Once a set of probes showing the suitable specificity and a set of control probes are provided, the latter are arranged in the array in a known position such that, after the steps of hybridization and of detection, it is possible to establish a correlation between a positive signal of hybridization and the particular gene from the coordinates of the array in which the positive signal of hybridization is detected.

The microarrays can be high density arrays with thousands of oligonucleotides by means of photolithographic in situ synthesis methods (Fodor et al., 1991, Science, 767-773). This type of probe is usually redundant, i.e., they include several probes for each mRNA which is to be detected. In a preferred embodiment, the arrays are low density arrays or LDA containing less than 10000 probes per square centimeter. In said low density arrays, the different probes are manually applied with the aid of a pipette in different locations of a solid support (for example, a crystal surface, a membrane). The supports used to fix the probes can be obtained from a large variety of materials, including plastic, ceramics, metals, gels, membranes, crystals and the like. The microarrays can be obtained using any methodology known for the person skilled in the art.

In the event that the expression levels of the genes according to the present invention is determined by measuring the levels of the polypeptide or polypeptides encoded by said gene or genes, the kits according to the present invention comprise reagents which are capable of specifically binding to said polypeptide or polypeptides. For this purpose, the arrays of antibodies such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like. The techniques for preparing said antibodies are very well known for the person skilled in the art and include the methods described by Ausubel et al. (Current Protocols in Molecular Biology, eds. Ausubel et al, John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, crystal or can be of a porous material as for example, acrylamide, agarose or another polymer. In another embodiment, it is possible to use cells producing the specific antibodies for detecting the proteins of the invention by means of their culture in array filters. After the induction of the expression of the antibodies, the latter are immobilized in the filter in the position of the array where the producing cell was located. An array of antibodies can be put into contact with a labeled target and the binding level of the target to the immobilized antibodies can be determined. If the target is not labeled, a sandwich type assay can be used in which a second labeled antibody specific for the polypeptide which binds to the polypeptide which is immobilized in the support is used. The quantification of the amount of polypeptide present in the sample in each point of the array can be stored in a database as an expression profile. The array of antibodies can be produced in duplicate and can be used to compare the binding profiles of two different samples.

In another aspect, the invention relates to the use of a kit of the invention for determination of the prognosis of a patient suffering from cancer or for the identification of a patient suffering from cancer which requires adjuvant therapy. In a preferred embodiment the cancer is lung or breast cancer.

The invention also relates to the use of
- a reagent specific for the determination of the expression levels of PGC1A, or
- a kit or assay device as described above
for predicting the outcome of a patient suffering from prostate cancer or for predicting the response to therapy of a patient suffering from prostate cancer.

All the terms and embodiments previously described are equally applicable to these aspects of the invention.

### EXAMPLES

The following invention is hereby described by way of the following examples, which are to be construed as merely illustrative and not limitative of the scope of the invention.

### Example 1: A bioinformatics screen identifies PGC1A as prognostic metabolic co-regulator in prostate cancer

Based on the complex combination of metabolic alterations that need to co-occur in order to ensure the metabolic rewiring in cancer, the inventors hypothesized that there must be a probabilistically parsimonious process that allows a major metabolic reprogramming with limited genetic alterations.

The inventors approached the study of prostate cancer metabolism applying criteria that ensure the selection of relevant master regulators contributing to the metabolic switch. They focused on transcriptional co-regulators of metabolism that i) were consistently altered in several publicly available datasets, and ii) were associated with reduced time to recurrence and disease aggressiveness.

The publicly available databases consulted have been published elsewhere (Cerami et al, Cancer Discov., 2012; Gao et al, Sci. Signal, 2013; Grasso et al, Nature, 2012; Lapointe et al, PNAS, 2004; Tomlins et al, Nat. Genet., 2007; Varambally et al, Cancer Cell, 2005; Rhodes et al, Neoplasia, 2004; Taylor et al, Cancer Cell, 2010; and Robinson et al, Cell, 2015).

The inventors first evaluated the expression levels of the metabolic co-regulators in a study comprising 150 prostate cancer specimens and 29 non-pathological prostate tissue. Figure 1a depicts the percentage of patients with up-regulation (dark grey) or down-regulation (light grey) of a given metabolic co-regulator (with a threshold of 2-fold difference in expression when compared to the mean expression in non-tumor prostate tissue). The analysis revealed 10 co-regulators in the set of study with significant differential expression in prostate cancer compared to normal tissue (Fig. 1a, asterisk). The inventors next extended this observation to four additional datasets in which there was available data for non-tumor and prostate cancer tissue (Grasso et al, Nature, 2012; Lapointe et al, PNAS, 2004; Tomlins et al, Nat. Genet., 2007; and Varambally et al, Cancer Cell, 2005). Only the alteration in PGC1A expression was further confirmed in the majority or all sets (Fig. 1b). Interrogation of the association of PGC1A with disease-free survival (DFS) and aggressiveness pointed at PGC1A as the sole co-regulator that exhibited a significant association with these parameters, which prompted us to continue the characterization of this transcriptional co-activator as the only candidate fulfilling the established criteria (Fig. 1c). Specifically, the inventors observed that the expression of PGC1A was decreased in association with increasing Gleason score and its down-regulation was significantly associated to shorter DFS in two independent datasets (Fig. 1c).

In order to rule out that cellular proliferation could contribute to the alteration of metabolic regulators, the inventors carried out an additional analysis in which they compared the expression of PGC1A in prostate cancer versus a benign hyperproliferative pathological alteration (benign prostate hyperplasia or BPH). The results corroborated that the decrease in PGC1A expression is associated to a cancerous state rather than to a proliferative condition.

The inventors observed that the expression of PGC1A was progressively decreased from primary tumors to metastasis (Fig. 1d). Strikingly, genomic analysis of PGC1A in prostate cancer revealed shallow deletions of the transcriptional co-activator (Fig. 1e). These genomic events were restricted to metastatic specimens, in full agreement with the notion that there is a selective pressure to reduce PGC1A expression as the disease progresses.

From the inventor's analysis, PGC1A emerges as the major master metabolic co-regulator altered in prostate cancer. The observed down-regulation and association with prostate cancer aggressiveness strongly suggests that it exerts a tumor suppressive activity.

### Example 2: Pgc1a deletion in the prostate epithelium promotes prostate cancer metastasis

PGC1A is a transcriptional co-activator that has been widely studied in the context of systemic metabolism, whereas its activity in cancer is just beginning to be understood. To date, there is no causal evidence in vivo of its role in cancer beyond the use of xenograft assays. To ascertain the role of PGC1A in prostate cancer initiation vs. progression, we conditionally deleted this metabolic co-regulator in the prostate epithelium alone or in combination with loss of the tumor suppressor Pten (Fig. 2a-c). Pgc1a deletion alone or in the context of Pten heterozygosity did not result in any differential tissue mass or histological alteration, which led us to conclude that it is not an initiating event (Fig. 2a, c). However, compound loss of both Pten and Pgc1a resulted in significantly larger prostate mass (Fig. 2b) together with a remarkable increase in the rate of invasive cancer (Fig. 2c). Histological analysis of the prostate revealed the existence of vascular invasion in double mutant mice (DKO), but not in Pten-deleted prostates. PGC1A has been reported to regulate the inflammatory response, which could influence and contribute to the phenotype observed. However, the inventors did not observe significant differences in the infiltration of polimorphonuclear netrophils (PMN) and lympho-plasmacytic infiltrates in their experimental settings. PGC1A has been also shown to induce the angiogenic process in coherence with the induction of VEGF-A expression in physio-pathological conditions and in cancer. In order to ascertain to which extent the hypothetical regulation of angiogenesis could explain the phenotype of our GEMM, the inventors analyzed VEGF-A expression and microvessel density. VEGF-A expression in GEMMs was unaffected by PGC1A deletion. In addition, microvessel density analysis through the quantification of CD31 immunoreactive vascular structures confirmed the lack of significant differences in these parameters in Pten^{Pc-/-} ; Pgc1a^{pc-/-} mice. The inventors therefore excluded the possibility that regulation of angiogenesis or inflammation downstream PGC1A could drive the phenotype characterized in this study.

Prostate cancer GEMMs faithfully recapitulate many of the features of the human disease. A reduced number of mouse models with clinically relevant mutations show increased metastatic potential. Strikingly, histopathological analysis of our mouse model in the context of Pten loss revealed that Pten^{pc-/-} ; Pgc1a^{pc-/-} mice - but not Pten^{pc-/-}; Pgc1a^{pc+/+} counterparts - presented evidence of metastasis, which was estimated in 44% to lymph nodes and 20% to liver (Fig. 2d). Metastatic dissemination was in agreement with the observation of PanCK and AR-positive Prostate cancer cell deposits in the lymph nodes of Pten^{pc-/-} ; Pgc1a^{pc-/-} mice. Of note, 33% of Pten^{pc-/-} ; Pgc1a^{pc+/+} mice presented small groups of PanCK-positive cells in LN (without metastatic lesions), suggesting that even if these cells are able to reach the LN, they lack capacity to establish metastatic lesions. Bone analysis revealed disseminated groups of PanCK-positive cells in Pten^{pc-/-} ; Pgc1a^{pc-/-} but not in Pten^{pc-/-} ; Pgc1a^{pc+/+} mice, although no clinical metastasis was detected, potentially owing to age of analysis (10-13 months), which was determined by humane endpoints. Analysis of a small cohort of Pten^{pc-/-} ; Pgc1a^{pc-/-} mice demonstrated that heterozygous loss of Pgc1a is sufficient to promote aggressiveness, vascular invasion and metastasis in a pre-established Prostate cancer setting. This observation supports the notion that single copy loss of PGC1A (as observed in metastatic human prostate cancer specimens, Fig. 1e) could be a key contributing factor to the metastatic phenotype.

The cooperative effect observed in our mouse model between loss of Pten and Pgc1a was supported by the direct correlation of the two transcripts in patient specimens and the association of PGC1A down-regulation with PTEN genomic loss.

In summary, the inventor's data in GEMMs and patient datasets formally demonstrates that the downregulation of PGC1A in Prostate cancer is an unprecedented causal event for the progression of the disease and its metastatic dissemination.

### Example 3: PGC1A suppresses prostate cancer growth and metastasis

In order to characterize the prostate tumor suppressive activity of PGC1A, the inventors first evaluated its expression level in well-established prostate cancer cell lines. Using previously reported PGC1A-positive and negative melanoma cells, they could demonstrate that prostate cancer cell lines lack detectable expression of the transcriptional co-activator at the protein level (Fig. 3a). In agreement with this notion, PGC1A-silencing in these cells failed to impact on the expression of its well-established targets. Importantly, through the analysis of publicly available datasets, we could demonstrate that the transcript levels of PGC1A in metastatic cell lines are comparable to those observed in metastatic Prostate cancer specimens and vastly reduced compared to PGC1A-positive melanoma cells (Fig. 3a). Of note, there are reports on PGC1A expression in prostate cancer cells. Despite their efforts to optimize the detection of the protein with different commercial antibodies, the inventors could not identify any immunoreactive band that would respond to PGC1A-targeting shRNAs nor would be coherent with PGC1A signal in melanoma cell lines expressing high levels of the protein. Yet, it cannot be ruled out that in non-basal conditions, stimulation of other factors such as androgen receptor or AMPK could lead to the up-regulation and allow detection of PGC1A in prostate cancer cells.

Due to the lack of PGC1A detection in Prostate cancer cellular systems, the inventors aimed at reconstituting the expression of this gene to levels achievable in the cancer cell lines previously reported. By means of lentiviral delivery of inducible PGC1A and doxycycline titration, they reached expression levels of this protein in three prostate cancer cell lines (Androgen receptor (AR) dependent - LnCaP - and independent - PC3 and DU145) equivalent to that observed in the PGC1A-expressing melanoma cell line MeWo (Fig. 3b). Next, we evaluated the cellular outcome of expressing PGC1A in prostate cancer cell lines. Interestingly, expression of PGC1A in this context resulted in a reduction in bi-dimensional and three-dimensional growth (Fig. 3c, d), cellular proliferation (Fig. 3e) and cell cycle progression. Of note, the inventors excluded the possibility that doxycycline treatment could influence the result of the growth analysis. This phenotype was recapitulated in vivo, where PGC1A expression decreased tumor formation and growth (Fig. 3f). In agreement with the GEMM data, they did not observe a contribution of angiogenesis to the phenotype.

The inventors observed in GEMMs that Pgc1a-loss results in metastatic dissemination (Fig. 2). They next sought to study whether PGC1A expression could oppose a pre-existing metastatic phenotype. To this end, they carried out xenotransplant assays in immunocompromised mice using luciferase-expressing PGC1A-inducible PC3 cells. Intra-cardial injection of these cells (Fig. 4a) revealed that PGC1A expression blunted metastatic growth in the lung, and led to a remarkable decrease in bone colonization (Fig. 4b-e).

Intra-cardial injection measures both dissemination and tumor re-initiation capacity. As an additional approach, the inventors sought to analyze metastatic tumor re-initiation capacity by means of local injection of prostate cancer cells at the metastatic site. Since prostate cancer exhibits osteotropic nature, we carried out an additional approach where cells were injected intra-tibially and the appearance of tumor masses in the bone was monitored (Fig. 4f). The results conclusively demonstrated that PGC1A exerts a potent anti-metastatic activity both in bone tumor mass and metastatic foci (Fig. 4g-h). These data provide conclusive evidence of the anti-proliferative and anti-metastatic potential of PGC1A.

### Example 4: The 10-gene metabomarker gene signature can be successfully used as a prognostic transcriptional signature for patient stratification

Additionally, the inventors surprisingly found that the transcriptional program regulated by PGC1A could serve to develop a prognostic gene signature.

Four large and well-annotated prostate cancer databases were used as starting material (Taylor et al, Cancer Cell, 2010; Grasso et al, Nature, 2012; Lapointe et al, PNAS, 2004; and TCGA (The Cancer Genome Atlas)).

In a first step, the Taylor and TCGA gene signatures were screened using the established starting criteria:
(i) Genes that are found down-regulated at higher frequency in prostate cancer than normal tissue (Fold >2)
(ii) Genes that are found down-regulated at higher frequency in tumors from patients with short recurrence (<24 months) (Fold >1)
(iii) Genes that are found down-regulated at higher frequency in tumors from patients with recurrence vs. Disease free (Fold>1).
(iv) Genes that exhibit a progressive down-regulation during prostate cancer progression.

*Downregulation was defined as expression below the mean of primary tumors in the dataset of interest.

Phase 1 of the first step: Genes in the Taylor dataset (N=179 genes) fulfilling any of (i) + (iii) or (ii) + (iii) were selected. The Taylor dataset relates to distribution and downregulated gene frequency in normal tissue, primary tumor and metastasis; and shows a correlation between downregulated gene frequency and time to recurrence; distribution and downregulated gene frequency in specimens from patients that will develop recurrence versus those that will remain disease free.

Phase 2 of the first step: Genes in the TCGA dataset (N=240 genes) fulfilling (iii) were selected. The TCGA dataset refers to recurrence and disease free survival.

In a second step, genes common to phase 1 and phase 2 were selected. Only genes complying with criteria (iv) were further evaluated. The Grasso (N=88; normal, primary tumor, mets) and Lapointe (N=26; normal, primary tumor, mets) datasets were used in a further step for validation (Figs. 5a and b).

A total of 10 genes were selected and this gene signature has been called metabomarker. It includes the genes:
- PGC1A
- ACACB
- ANG
- ATP5L
- GSTM4
- NDUFA4
- NNT
- PRKRA
- RNASE4
- TP53INP2

The prognostic potential of the metabomarker gene signature has been established against the full Taylor and TCGA databases (Fig. 5c-d). In regards the Taylor dataset (Fig. 5c), the hazard ratio (HR) of patients exhibiting low average signal of the gene signature (first quartile, lower 25%, Q1) versus patients with high average signal of the gene signature (Rest, quartiles 2-4) is 3.06, whereas the HR of patients with high average signal of the gene signature (Rest, quartiles 2-4) versus patients exhibiting low average signal of the gene signature (first quartile, lower 25%, Q1) is 0.32. In regards the TCGA dataset (Fig. 5d), the HR of patients exhibiting low average signal of the gene signature (first quartile, lower 25%, Q1) versus patients with high average signal of the gene signature (Rest, quartiles 2-4) is 11.5, whereas the HR of patients with high average signal of the gene signature (Rest, quartiles 2-4) versus patients exhibiting low average signal of the gene signature (first quartile, lower 25%, Q1) is 0.08.

### Example 5: The 10-gene metabomarker gene signature can be successfully used to predict response to therapy

Finally, the inventors surprisingly found that the transcriptional program regulated by PGC1A could be used to predict response to therapy in a patient suffering from prostate cancer.

Thus, prostate cancer cells expressing PGC1A are better suited to withstand aggressive cytotoxic insults, such as hydrogen peroxide (Fig. 6a, b) and they fare better when glucose availability is reduced (Fig. 6c) compared to cancer cells not expressing PCG1A.

### Materials and Methods

### Reagents

3-[4-(2,4-Bis-trifluoromethylbenzyloxy)-3-methoxyphenyl]-2-cyano-N-(5-trifluoromethyl-1,3,4-thiadiazol-2-yl) acrylamide (XCT 790), etomoxir (ETO), Doxycycline hyclate (Dox), oligomycin, Nacetyl-cysteine (NAC) and Manganese (III) tetrakis (4-benzoic acid)porphyrin chloride (MnTBAP) were purchased from Sigma.

### Cell culture

Human prostate carcinoma cell lines, LnCaP, DU145 and PC3 were purchased from Leibniz-Institut DSMZ - Deutsche Sammlung von Mikroorganismen und Zellkulturen GmbH, who provided authentication certificate. Cells were transduced with a modified TRIPZ (Dharmacon) doxycycline inducible lentiviral construct in which the RFP and miR30 region was substituted by HA-Flag-Pgc1a or HA-Flag-Pgc1aL2L3M. Lentiviral shRNA constructs targeting PGC1A where purchased in Sigma (TRCN0000001166) and a scramble shRNA was used as control. Melanoma lines were kindly provided by Dr. Boyano and Dr. Buque and purchased from ATCC. Cell lines were routinely screened for mycoplasma contamination and quarantined while treated if positive.

### Animals

All mouse experiments were carried out following the ethical guidelines established by the Biosafety and Welfare Committee at CIC bioGUNE and The Institutional Animal Care and Use Committee of IRB Barcelona. The procedures employed were carried out following the recommendations from AAALAC. Xenograft experiments were performed, injecting 10⁶ cells per condition in two flanks per mouse. PC3 TRIPZ-HA-Pgc1a cells were injected in each flank of nude mice and 24 h post-injections mice were fed with chow or doxycycline diet (Research diets, D12100402). GEMM experiments were carried out in a mixed background (where the founder colony was cross-bred for at least three generations prior to the expansion of experimental cohorts in order to ensure and homogenous mixed background). The Pten^{loxP} and Pgc1a^{loxP} conditional knockout alleles have been described elsewhere (Chen et al, Nature, 2005; and Lin et al, Cell, 2004). Prostate epithelium specific deletion was effected by the Pb-Cre4. Mice were fasted for 6 h prior to tissue harvest (9 am-3 pm) in order to prevent metabolic alterations due to immediate food intake.

For intra-tibial and intra-cardiac injections BALB/c nude male mice (Harlan) of 9-11 weeks of age were used. Before the injections, PC3 PGC1 cell line was pre-treated for 48h with PBS or doxycycline (0.5µg/ml). Mice injected with cells treated with doxycycline were also pre-treated for 48h with 1mg/ml of doxycycline in drinking water. After the injections this group of mice was left on continuous doxycycline treatment (1mg/ml in drinking water). Before the injections mice were anesthetized with mixture of Kethamine (80 mg/kg) and Xilacine (8 mg/kg). For intra-tibial injections, 1x10⁴ cells were resuspended in final volume of 5 µl of cold PBS and injected as described previously. For intra-cardiac injections 2x10⁵ cells were resuspended in final volume of 100 µl of cold PBS and injected as described previously. Upon the injections tumor development was followed on weekly basis by BLI using the IVIS-200 imaging system from Xenogen. Quantification of bioluminescent images was done with Living Image 2.60.1 software. Upon necropsy, the development of metastasis was confirmed by doing ex vivo bioluminescent images of organs of interest as well as in histological sections. For metastasis-free survival curves metastatic event was scored when measured value of bioluminescence bypasses 1/10 of the day 0 value.

### Patient samples,

All samples were obtained from the Basque Biobank for research (BIOEF, Basurto University hospital) upon informed consent and with evaluation and approval from the corresponding ethics committee (CEIC code OHEUN11-12 and OHEUN14-14).

### Cellullar Molecular and metabolic assays

Cell number quantification with crystal violet and western blot were performed using protocols well known to a person skilled in the art.

RNA was extracted using NucleoSpin® RNA isolation kit from Macherey-Nagel (ref: 740955.240C). For patients and animal tissues a Trizol-based implementation of the NucleoSpin® RNA isolation kit protocol was used. For all cases, 1µg of total RNA was used for cDNA synthesis using qScript cDNA Supermix from Quanta (ref. 95048). Quantitative Real Time PCR (q-RTPCR) was performed. Universal Probe Library (Roche) primers and probes employed are detailed in table 1. β-ACTIN (Hs99999903-ml; Mm0607939_s1) and GAPDH (Hs02758991_gl, Mm99999915_g1) housekeeping assays from Applied Biosystems showed similar results (all qRT-PCR data presented was normalized using GAPDH/Gapdh).

FAO was performed using protocols well known to a person skilled in the art. Lactate production was performed using Trinity Biotech lactate measurement kit. Oxygen consumption rate (OCR) was measured with a XF24 extracellular flux analyzer (Seahorse Bioscience). Briefly, 50.000 cells per well were seeded in a XF24 plate, and OCR measurements were normalized to cell number analyzed by crystal violet. Cells were initially plated in 10% FBS DMEM media for 24 hours, and 1h before measurements, media was changed to DMEM serum and bicarbonate free, with glutamine and glucose (10mM). Mitochondrial stress test was carried out using the following concentrations of injected compound: Oligomycin (1µM).

For mitochondrial ATP assays, 50.000 PC3 and DU145 cells plated onto 13-mm coverslips and transfected with a mitochondrial targeted luciferase chimera (mtLuc). Cells were perfused in the luminometer at 37°C with KRB solution containing 25 µM luciferin and 1 mM CaCl₂ and supplemented with 5.5 mM glucose. Under these conditions, the light output of a coverslip of transfected cells was in the range of 5.000-20.000 cps for the luciferase construct vs. a background lower than 100 cps. Luminescence was entirely dependent on the presence of luciferin and was proportional to the perfused luciferin concentration between 20 and 200 µM.

Mitochondrial morphology was assessed by using a cDNA encoding mitochondrial matrixtargeted DsRed (mtDsRed). Cells were seeded onto 24-mm diameter coverslip (thickness between 0.16-0.19 mm) (Thermo Scientific) and 24 h later cells were transfected with 2 µg mtDSred (Lipofectamine LTX reagent; Invitrogen). mtDsRed expression was assessed 36 h later. All the acquisitions were performed with a confocal Nikon Eclipse Ti system and fluorescent images were captured by using NisElements 3.2.

Lipid peroxidation based on MDA detection was assayed in xenograft samples following the manufacture instructions (MAK085 Sigma-Aldrich).

ROS production was determined by Mitosox and DCF staining.

**Table 1. List of qRT-PCR primers**

| **Gene** | **Species** | **Forward Sequence** | **Reverse Sequence** | **Probe** |
|---|---|---|---|---|
| ACACB | human | cagacgctacaggtcccaac (SEQ ID NO:1) | ctgtccactccactgtcagg (SEQ ID NO:2) | 37 |
| Acacb | mouse | tgaatctcacgcgcctacta (SEQ ID NO:3) | ttgtgttctcggcctctctt (SEQ ID NO:4) | 107 |
| ACADM | human | aggagccattgatgtgtgc (SEQ ID NO:5) | ctgctttggtctttataccagcta (SEQ ID NO:6) | 1 |
| ACAT1 | human | gatccccaaaaagtgaatatcaa (SEQ ID NO:7) | atcctggctccagacatcc (SEQ ID NO:8) | 17 |
| Acat1 | mouse | agggaagtttgccagtgaga (SEQ ID NO:9) | ttcaccaccacatctggtttac (SEQ ID NO:10) | 9 |
| ACO2 | human | gtgtagactccatctcctgcact (SEQ ID NO:11) | tgtggttgtaagggaacacg (SEQ ID NO:12) | 49 |
| ACSL4 | human | tgtactgtactgaagcccacactt (SEQ ID NO:13) | ttcatctcttggactttgctca (SEQ ID NO:14) | 66 |
| Acsl4 | mouse | gaaattcacagcatgcaatcag (SEQ ID NO:15) | tctacttggaggaacgctcaa (SEQ ID NO:16) | 17 |
| ATP1B1 | human | cggtggcagttggtttaag (SEQ ID NO:17) | agcatcacttggatggttcc (SEQ ID NO:18) | 20 |
| CLYBL | human | gcccagaacactgttacgc (SEQ ID NO:19) | tgcagcttcaataccctttagc (SEQ ID NO:20) | 10 |
| ETFDH | human | cccgggataaggacaagag (SEQ ID NO:21) | catctgcttcttctgcaaacc (SEQ ID NO:22) | 12 |
| FH | human | gcacagatcatcaagattggac (SEQ ID NO:23) | ttgttgaacataaccactaaattcct (SEQ ID NO:24) | 89 |
| Fh1 | mouse | gcaccccaatgatcatgtta (SEQ ID NO:25) | attgctgtgggaaaggtgtc (SEQ ID NO:26) | 106 |
| GOT1 | human | agctgtgcttctcgtcttgc (SEQ ID NO:27) | agattgcacacctcctaccc (SEQ ID NO:28) | 26 |
| GSTM4 | human | tgctacagccctgactttga (SEQ ID NO:29) | tgatcttgtctccaacaaaccat (SEQ ID NO:30) | 60 |
| HADHA | human | caccctcactgcgctagac (SEQ ID NO:31) | ttcttcactttgtcattcaatcct (SEQ ID NO:32) | 56 |
| IDH3 | human | tttttgatgctgccaaagc (SEQ ID NO:33) | ttcctccaggtccttgaatg (SEQ ID NO:34) | 52 |
| Idh3a | mouse | gaggttttgctggtggtgtt (SEQ ID NO:35) | tgaaatttctgggccaattc (SEQ ID NO:36) | 80 |
| ISCU | human | aacacagatatcgccaaggag (SEQ ID NO:37) | attgcatcttcagccagcat (SEQ ID NO:38) | 8 |
| LAMB2 | human | ctggtggcagtcagagaatg (SEQ ID NO:39) | cagcagggcgaaatgtct (SEQ ID NO:40) | 42 |
| MPC1 | human | gcggcttatcaaacacgag (SEQ ID NO:41) | agggaggctatttataatgaaatctg (SEQ ID NO:42) | 56 |
| MPC2 | human | aaaattggagtctgtttgctgtt (SEQ ID NO:43) | tgtgctttagcttttagttcttgg (SEQ ID NO:44) | 18 |
| PGC1A | mouse | gaaagggccaaacagagaga (SEQ ID NO:45) | gtaaatcacacggcgctctt (SEQ ID NO:46) | 29 |
| PGC1A | human | tgagagggccaagcaaag (SEQ ID NO:47) | ataaatcacacggcgctctt (SEQ ID NO:48) | 13 |
| Pten | mouse | tccacaaacagaacaagatgct (SEQ ID NO:49) | ccattttccactttttctgagg (SEQ ID NO:50) | 93 |
| SDHA | human | tccactacatgacggagcag (SEQ ID NO:51) | ccatcttcagttctgctaaacg (SEQ ID NO:52) | 70 |
| Sdha | mouse | tgttcagttccaccccaca (SEQ ID NO:53) | tctccacgacacccttctg (SEQ ID NO:54) | 71 |
| SUCLA | human | gttctacggcaggctagtgg (SEQ ID NO:55) | acaatccagaacttcccagaac (SEQ ID NO:56) | 66 |
| Sucla | mouse | tccgatgaagcttacgcaat (SEQ ID NO:57) | tgtaaatgttccttttcctctgc (SEQ ID NO:58) | 98 |
| TP53INP2 | human | ccttgaagtcctagagtccaataaa (SEQ ID NO:59) | ctatggcagtgcaaaaacctc (SEQ ID NO:60) | 16 |
| Vegfa | mouse | caggctgctgtaacgatgaa (SEQ ID NO:61) | gctttggtgaggtttgatcc (SEQ ID NO:62) | 9 |
| VEGFA | human | cctccgaaaccatgaacttt (SEQ ID NO:63) | atgattctgccctcctcctt (SEQ ID NO:64) | 22 |

### Histopathological analysis

After euthanasia, histological evaluation of a Hematoxylin and eosin (H&E) stained section from formalin-fixed paraffin embedded tissues of the following organs was performed: prostate gland, lymph nodes, long bones from lower limbs and other solid organs such as lungs, liver, spleen and kidneys.

Following the consensus reported by Ittmann et al, Cancer Res., 2007, prostate gland alterations were classified into 4 categories: gland within normal limits; high grade prostatic intraepithelial neoplasia (HGPIN); HGPIN with focal micro-invasion; and invasive carcinoma. Lymphovascular invasion was assessed in all cases were micro-invasion foci or invasive carcinoma were observed.

Lymph node (LN) metastasis and the presence of groups of Prostate cancer cells in bone marrow (BM) were determined after hematoxylin-eosin (H&E) staining (LN) and immunohistochemical identification of cytokeratin (CK) and androgen receptor (AR) - expressing cells using a pan-CK rabbit polyclonal antibody (Dako, Carpinteria, CA) and AR rabbit polyclonal antibody (Santa Cruz Biotechnology, sc-816) (LN and BM). In the case of BM, cases were classified as "dissemination negative" when none or few scattered (less than 5) CK-expressing cells were identified and "dissemination positive" when more than 5 or small groups of these cells were observed.

To assess the inflammatory component in the prostate tissues we performed a semiquantitative analysis in the glandular and the stromal areas separately for each of the specimens. We first determined the type of inflammatory cell present in each tissue compartment: polymorphonuclear neutrophils versus lympho-plasmacytic infiltrates. Then we performed a quantification of these cells using the following score system: 0-no inflammatory cells, 1-few cells, 2-moderate amount of cells and 3-high amount of cells. Scores in between were also determined as 0.5, 1.5 and 2.5. If both types of cells were present in one compartment, we chose the highest as the final score.

Histological features of the generated tumor xenografts were evaluated on H&E stained sections from formalin-fixed paraffin embedded tissues. Proliferation was assessed in paraffin embedded xenografts samples by using Ki67 antibody (MA5-14520, Thermo Scientific). Microvessel density was determined and quantified in GEMMs and xenograft samples by the immunodetection of CD31 (Rabbit anti-CD31; Ref. ab28364 Abcam).

### Metabolomics

LCHR-MS metabolomics and stable isotope 13C-U6-Glucose labelling was performed using protocols well known to a person skilled in the art. Briefly, PC3 TRIPZ-HA-Flag-Pgc1a cells treated or untreated for 72h with 0.5 µg/ml doxycycline were plated at 500.000 cells/well in 6-well plates. For LCHR-MS metabolomics and grown maintaining the doxycycline regime for 42h before harvesting, while for stable isotope 13C-U6-Glucose labelling experiments, 24h after seeding cells were washed and exposed to media with serum, without glucose and pyruvate and supplemented 2mM 13C-U6-Glucose. 16h after that, cells were washed and another 13C-U6-Glucose pulse was performed for 2h before harvesting.

### Transcriptomic analysis

For transcriptomic analysis in PC3 TRIPZ-HA-Flag-PGC1A cells, Illumina whole genome -HumanHT- 12_V4.0 (DirHyb, nt) method was used.

Promoter enrichment analysis was assessed with the Transcription Factors (TFs) dataset from MSigDB (The Molecular Signature Database; http://www.broadinstitute.org/gsea/msigdb/collections.jsp). TFs dataset contain genes that share a transcription factor-binding site defined in the TRANSFAC (version 7.4, http://www.generegulation.com/) database. Each of these gene signatures was annotated by a TRANSFAC record. A hypergeometric test was used to detect enriched dataset categories.

The GSEA was performed using the GenePattern web tool from the Broad Institute (http://genepattern.broadinstitute.org). The list of PGC1A upregulated genes ranked by their fold change was uploaded and analyzed against a list of ERRα target genes. The number of permutations carried out were 1000 and the threshold was 0.05.

### Bioinformatic analysis and statistics

Database normalization: all the datasets used for the data mining analysis were downloaded from GEO, and subjected to background correction, log2 transformation and quartile normalization. In the case of using a pre-processed dataset, this normalization was reviewed and corrected if required.

Frequency of alteration of metabolic co-regulators: expression levels of the selected co-regulators were obtained from the dataset reported by Taylor et al, Cancer Cell, 2010. A matrix containing signal values and clinical information was prepared in order to ascertain the up- or downregulation. The inventors computed the relative expression of an individual gene and tumor to the expression distribution in a reference population (patients without prostate tumor or metastasis). The returned value indicates the number of standard deviations away from the mean of expression in the reference population (Z-score). Using a fold change of +2 and -2 as a threshold, the number of samples from the cancer dataset that were up- or down-regulated was determined. p-values were calculated by comparing the means of normal of cancerous biopsies.

Quartile analysis in DFS: Patients biopsies from primary tumors were organized into four quartiles according to the expression of the gene of interest. The recurrence of the disease was selected as the event of interest. Kaplan-Meier estimator was used to perform the test as it takes into account right-censoring, which occurs if a patient withdraws from a study. On the plot, small vertical tick-marks indicate losses, where a patient's survival time has been right-censored. With this estimator we obtained a survival curve, a graphical representation of the occurrence of the event in the different groups, and a p-value that estimate the statistical power of the differences observed.

PGC1A target gene list analysis in patient datasets, genes of interest in each patient were expressed as 1 (down-regulated) or 0 (not down-regulated). The mean of the gene expression in the primary tumor group (mean PT) of patients was calculated. After that, the expression of the gene in each patient was compared to this value to define whether it was lower, in which case it was represented with a 1. 0 was assigned to genes in patients not showing down-regulation. In the Taylor dataset, for genes represented by multiple transcripts, down-regulation (value = 1) was considered when at least 50% of the probes were categorized as 1.

For PGC1A genomic analysis, data from prostate cancer patients with copy number alteration information in Taylor, Grasso and Robinson datasets was extracted from cbioportal.org. Percentage of shallow deletions of primary tumors and metastatic patients was calculated separately.

Correlation analysis: Pearson correlation test was applied to analyze the relation between paired genes. From this analysis, Pearson's coefficient (R) indicates the existing linear correlation (dependence) between two variables X and Y, giving a value between +1 and -1 (both included), where 1 is total positive correlation, 0 is no correlation, and -1 is total negative correlation. The p-value indicates the significance of this R coefficient.

Statistical analysis: Data represent mean ± s.e.m. of pooled experiments unless otherwise stated. n values represent the number of independent experiments performed or the number of individual mice. For each independent in vitro experiment, at least three technical replicates were used (exceptions: in western blot analysis technical replicates are presented, in untargeted metabolomics two technical replicates were used and for, 13C-U6-Glucose isotope labeling one technical replicate was used) and a minimum number of three experiments were done to ensure adequate statistical power. For data mining analysis, ANOVA test was used for multi-component comparisons and Student T test for two component comparisons. In the in vitro experiments, data groups were assessed for normal distribution and Student T test was applied for two component comparisons. For in vivo experiments, equal variance could not be assumed and a non-parametric Mann-Whitney exact test was used, without using approximate algorithms to avoid different outcomes of statistics packages (Bergmann R. Statistical Computing and Graphics).

For the analysis of PGC1A signature frequency in prostate cancer human specimens, a Chi Square test was performed. The confidence level used for all the statistical analyses was of 0.95 (alpha value = 0.05). Two-tail statistical analysis was applied for experimental design without predicted result, and one-tail for validation experiments.

## Claims

1. A method for predicting the outcome of a patient suffering from prostate cancer, which comprises the determination of the expression level of PGC1A in a sample from the patient, wherein
a) an increase in the expression level of PGC1A with respect to a reference value is indicative of a decreased risk of negative outcome of the patient; or wherein
b) a decrease in the expression level of PGC1A with respect to a reference value is indicative of an increased risk of negative outcome of the patient.

2. A method for predicting the outcome of a patient suffering from prostate cancer, which comprises the determination of the average expression levels of the PGC1A, ACACB, ANG, ATP5L, GSTM4, NDUFA4, NNT, PRKRA, RNASE4, and TP53INP2 genes in a sample from the patient, wherein
a) an increased average expression level of said genes with respect to a reference value is indicative of a decreased risk of negative outcome of the patient; or
b) a decreased average expression level of said genes with respect to a reference value is indicative of an increased risk of negative outcome of the patient.

3. The method of any of claims 1 or 2 wherein the outcome of the patient is determined as time to cancer recurrence, overall survival, disease-specific survival, disease-free survival or occurrence of metastasis.

4. A method for predicting the response to therapy of a patient suffering from prostate cancer, which comprises the determination of the expression level of PGC1A in a sample from the patient, wherein
a) an increase in the expression level of PGC1A with respect to a reference value is indicative of a positive response of the patient to therapy; or wherein
b) a decrease in the expression level of PGC1A with respect to a reference value is indicative of a negative response of the patient to therapy.

5. A method for predicting the response to therapy of a patient suffering from prostate cancer, which comprises the determination of the average expression level of the PGC1A, ACACB, ANG, ATP5L, GSTM4, NDUFA4, NNT, PRKRA, RNASE4, and TP53INP2 genes in a sample from the patient, wherein
a) an increased average expression of said genes with respect to a reference value is indicative of a positive response of the patient to therapy; or
b) a decreased average expression of said genes with respect to a reference value is indicative of a negative response of the patient to therapy.

6. A method for selecting a patient to be treated for advanced, recurrent or metastatic prostate cancer which comprises the determination of the expression level of PGC1A in a sample from the patient, wherein a decrease in the expression level of PGC1A with respect to a reference value is indicative that the patient is selected for treatment for advanced prostate cancer.

7. A method for selecting a patient to be treated for advanced, recurrent or metastatic prostate cancer which comprises the determination of the average expression level of the PGC1A, ACACB, ANG, ATP5L, GSTM4, NDUFA4, NNT, PRKRA, RNASE4, and TP53INP2 genes in a sample from the patient, wherein a decreased average expression of said genes with respect to a reference value is indicative that the patient is selected for treatment for advanced prostate cancer.

8. The method of any of claims 1, 4 or 6 wherein the reference value is the mean level of expression of PGC1A in a pool of samples from primary prostate tumors.

9. The method of any of claims 2, 5 or 7 wherein the reference value is the mean level of the average expression of the PGC1A, ACACB, ANG, ATP5L, GSTM4, NDUFA4, NNT, PRKRA, RNASE4, and TP53INP2 genes in a pool of samples from primary prostate tumors.

10. The method of any of claims 1 to 9 wherein the sample is a sample containing tumor cells.

11. The method of claim 10 wherein the sample containing tumor cells is tumor tissue.

12. The method of claim 11 wherein tumor tissue is a tumor biopsy or a surgically resected tumor.

13. The method of any of claims 1 to 12 wherein the patient is a patient in which a primary prostate tumor has been surgically excised.

14. The method of any of claims 1 to 13 wherein the determination of the expression levels of the gene or genes is carried out by determining the levels of the corresponding mRNAs or by determining the levels of the polypeptides encoded by said genes.

15. A method of treatment of advanced, recurrent or metastatic prostate cancer to a patient in need thereof which comprises the administration of a therapy to said patient, wherein the patient is selected by a method of any of claims 6 to 14.

16. A kit or assay device comprising reagents adequate for the determination of the average expression level of the PGC1A, ACACB, ANG, ATP5L, GSTM4, NDUFA4, NNT, PRKRA, RNASE4, and TP53INP2 genes, wherein the reagents are selected from the group of a set probes which specifically hybridize to the mRNA of said genes and a set of primer pairs which are capable of specifically amplifying the mRNAs of said genes and wherein said reagents comprise at least 10% of the reagents present in the kit..

17. A kit or assay device comprising reagents adequate for the determination of the average expression level of the polypeptides encoded by the PGC1A, ACACB, ANG, ATP5L, GSTM4, NDUFA4, NNT, PRKRA, RNASE4, and TP53INP2 genes wherein the reagents are a set of antibodies which specifically bind to the polypeptides encoded by said genes and wherein said reagents comprise at least 10% of the reagents present in the kit.

18. Use of
- a reagent specific for the determination of the expression levels of PGC1A, or
- a kit or assay device according to any one of claims 16 or 17
for predicting the outcome of a patient suffering from prostate cancer or for predicting the response to therapy of a patient suffering from prostate cancer.
